# EUROPEAN PATENT APPLICATION

(11) **EP 2 034 026 A1**
(43) Date of publication of application: **11.03.2009**
(21) Application number: 07744682.1
(22) Date of filing: 05.06.2007
(51) Int. Cl.: C12P 7/04, C12N 15/00

(54) **PROCESS FOR PRODUCTION OF OPTICALLY ACTIVE ALCOHOL**

(30) Priority: 05.06.2006 JP 2006156059
(71) Applicant: DAICEL CHEMICAL INDUSTRIES, LTD., Osaka-shi, Osaka 530-0001 (JP)
(72) Inventor: HAYASHI, Motoko c/o DAICEL CHEMICAL INDUSTRIES, LTD, Himeji-shi, Hyogo 671-1283 (JP); NIKAIDO, Teruyuki c/o DAICEL CHEMICAL INDUSTRIES, LTD, Himeji-shi, Hyogo 671-1283 (JP); YAMAMOTO, Hiroaki c/o DAICEL CHEMICAL INDUSTRIES, LTD, Himeji-shi, Hyogo 671-1283 (JP)
(74) Representative: Grünecker, Kinkeldey, Stockmair & Schwanhäusser Anwaltssozietät
(86) International application number: PCT/JP2007/061325
(87) International publication number: WO 2007/142210

(57) **Abstract**

The present invention provides methods for producing (S)-1,1,1-trifluoro-2-propanol, which include the step of reacting an enzyme of any one of alcohol dehydrogenase CpSADH, alcohol dehydrogenase ReSADH, carbonyl reductase ScoPAR, (2S,3S)-butanediol dehydrogenase ZraSBDH, carbonyl reductase ScGCY1, tropinone reductase HnTR1, tropinone reductase DsTR1, or alcohol dehydrogenase BstADHT, a microorganism or a transformant strain that functionally expresses the enzyme, or a processed material thereof, with 1,1,1-trifluoroacetone. The present invention also provides methods for producing (R)-1,1,1-trifluoro-2-propanol, which include the step of reacting alcohol dehydrogenase PfODH, a microorganism or a transformant strain that functionally expresses the enzyme, or a processed material thereof, with 1,1,1-trifluoroacetone.

SEQUENCE LISTING

<110>  DAICEL CHEMICAL INDUSTRIES, LTD.

<120>  PROCESS FOR PRODUCTION OF OPTICALLY ACTIVE ALCOHOL

<130>  EP62893HVpau

<140>  EP 07 744 682.1
<141>  2007-06-05

<150>  PCT/JP2007/061325
<151>  2007-06-05

<150>  JP 2006-156059
<151>  2006-06-05

<160>  38

<170>  PatentIn version 3.3

<210>  1
<211>  1011
<212>  DNA
<213>  Candida parapsilosis

<400>  1
   atgtcaattc catcaagcca gtacggattc gtattcaata agcaatcagg acttaatttg      60
   cgcaatgatt tgcctgtcca caagcccaaa gcgggtcaat tgttgttgaa agttgatgct     120
   gttggattgt gtcattctga tttacatgtc atttacgaag ggttggattg tggtgataat     180
   tatgtaatgg gacatgaaat tgctggaact gttgctgctg tgggtgatga tgtcattaac     240
   tacaaggttg gtgatcgtgt tgcctgtgtc ggacccaatg gatgtggtgg gtgcaagtat     300
   tgtcgtggtg ccattgacaa tgtatgtaaa aacgcatttg gtgattggtt cggattgggg     360
   tacgatggtg ggtatcaaca gtacttgttg gttactcgcc cacgtaactt gtctcgtatc     420
   ccagataacg tatctgcaga cgtggctgcg gcttcaactg atgctgtatt gacaccatat     480
   cacgcaatca agatggctca agtgtcacca acttcgaata tcttgcttat tggtgctggt     540
   ggattgggtg gaaatgcaat tcaagttgcc aaggcatttg gtgcgaaagt tactgttttg     600
   gacaaaaaaa aggaggctcg tgaccaagca aagaagttgg gtgctgatgc agtttatgaa     660
   acattgccag aatccatttc tcctggctct ttttcagcat gttttgattt tgtttcagtg     720
   caagctacat ttgatgtatg tcaaaagtat gttgaaccaa agggtgtaat tatgcccgtg     780
   ggactcggtg ctcctaattt atcgtttaat ttgggagatt tggcattgcg cgaaattcga     840
   atcttgggta gtttttgggg aactactaat gatttggatg atgttttgaa attggttagt     900
   gaaggtaaag ttaaacccgt tgtgcgcagt gccaaattga aggaattgcc agagtatatt     960
   gaaaaattgc gcaacaatgc ttatgaaggt cgtgttgttt ttaatccata a             1011

<210>  2
<211>  1047
<212>  DNA
<213>  Rhodococcus erythropolis

<400>  2
atgaaagcaa tccagtacac gagaatcggc gcggaacccg aactcacgga gattcccaag      60

cccgagcccg gtccaggtga agtgctcctg gaagtcaccg ctgccggcgt ctgccactcg 120
gacgacttca tcatgagcct gcccgaagag cagtacacct acggccttcc gctcacgctc 180
ggccacgaag gcgcaggcaa ggtcgccgcc gtcggcgagg gtgtcgaagg tctcgacatc 240
ggaaccaatg tcgtcgtcta cgggccttgg ggttgcggca actgttggca ctgctcacaa 300
ggactcgaga actattgctc tcgcgcccaa gaactcggaa tcaatcctcc cggtctcggt 360
gcacccggcg cgttggccga gttcatgatc gtcgattctc ctcgccacct tgtcccgatc 420
ggtgacctcg acccggtcaa gacggtgccg ctgaccgacg ccggtctgac gccgtatcac 480
gcgatcaagc gttctctgcc gaaacttcgc ggaggctcgt tcgcggttgt cattggtacc 540
ggcggtctcg gccacgtcgc tattcagctc ctccgtcacc tctcggcggc aacggtcatc 600
gctttggacg tgagcgcgga caagctcgaa ctggcaacca aggtaggcgc tcacgaagtg 660
gttctgtccg acaaggacgc ggccgagaac gtccgcaaga tcactggaag tcaaggcgcc 720
gcactggttc tcgacttcgt cggctaccag cccaccatcg acaccgcgat ggctgtcgcc 780
ggcgtcggat cagacgtcac gatcgtcggg atcggggacg gccaggccca cgccaaagtc 840
gggttcttcc aaagtcctta cgaggcttcg gtgacagttc cgtattgggg tgcccgcaac 900
gagttgatcg aattgatcga cctcgcccac gccggcatct tcgacatcgc ggtggagacc 960
ttcagtctcg acaacggtgc cgaagcgtat cgacgactgg ctgccggaac gctaagcggc 1020
cgtgcggttg tggtccctgg tctttaa 1047

<210> 3
<211> 1041
<212> DNA
<213> Streptomyces coelicolor

<400> 3
atgaaagcac tgcagtatcg tactattggt gcaccacctg aagttgtaac ggtaccagat 60
ccagaacctg gtccaggtca agttttactt aaagttactg cggccggtgt gtgccatagc 120
gatatcgctg ttatgtcttg gcctgccgaa ggcttcccgt atgaactgcc gctgaccctg 180
ggtcacgagg gcgtaggtac tgtggcagcg ctgggcgccg gcgttaccgg cctggcagaa 240
ggcgacgcgg tcgctgtgta tggtccgtgg ggctgtggta cgtgcgccaa atgtgcagaa 300
ggcaaagaaa attattgcct gcgtgcggat gagctgggga tccgccctcc gggtcttggc 360
cgtccgggtt ctatggctga atacctgctc atcgacgatc cgcgccatct cgtgccgctg 420
gatggcttag atccggtagc agcggtgccg ctgaccgacg ctggtttgac tccgtaccac 480
gccattaaac gttcactgcc taagctggtt ccggggtcga ccgcagtcgt gatcgggact 540
ggtggccttg gtcatgttgc gatccaattg ctccgcgctc tgacgtcagc gcgtgtagtg 600
gcgctggatg tcagcgaaga aaagttacgc ctggcacgtg ccgtcggcgc acacgaggcg 660
gtgttgtctg acgctaaagc agcggatgct gttcgcgaaa ttaccggtgg cctgggggcc 720
gaagcagtat tcgattttgt gggtgttgcg ccaaccgtcc agaccgcggg cgcagtggcg 780
gctgttgaag gtgacgtaac cctggtgggc atcggtggcg ggtccttgcc ggttggtttt 840

ggcatgctgc cgttcgaagt ctcagtgaac gccccgtatt ggggttcgcg tagtgaactc 900

actgaagttc tgaacctggc acgcagcggc gcggtatctg tacataccga aacgtactcc 960

ttagatgacg ctccgctggc gtacgagcgc ttgcacgaag gtcgcgttaa tggtcgtgct 1020

gttattttac cacatggtta a 1041

<210> 4
<211> 780
<212> DNA
<213> Zoogloea ramigera

<400> 4
atgtcgttga atggcaaagt cattttggta accggcgctg gccaggggat cggacgcggt 60

attgcgctgc ggcttgcaaa ggaaggcgct gatctcgcgc tggccgacgt caaggccgat 120

aagctcgact ccgttcgcaa ggaagtcgaa gcgctcggac gcaaggccac caccgtcgtc 180

gccgatgtca gcaagcgcga cgaagtctac gccgccatcg accatgcaga gaagcaactc 240

ggcgggttcg acgtcatggt caacaatgcc ggcatcgccc aggtcaagcc gatcgccgac 300

gtcacgcccg aggacatgga cctgatcttc cggatcaatg tcgacggcgt gctctggggc 360

atccaggcgg cttcgcagaa attcaaggat cgaggtcaga agggcaagat catcaatgcc 420

tgctcgattg ccggacatga cggcttcgcc atgctcggcg tctattcggc aaccaagttc 480

gccgtgcgtg cgctgacgca agccgccgcc aaggaatatg ccagcgcggg catcacggtg 540

aacgcctact gccccggcat cgtcggcacc gacatgtggg tggagatcga cgagcgcttt 600

tccgagatca ccggaacgcc gaagggcgaa acctacaaga aatacgtcga gggcatcgct 660

ttgggccgcg cgcagacacc ggaggacgtg gcagcgttgg tcgccttcct cgcgggcgcc 720

gattccgact acatcacggg acagtcgatc ctgaccgatg gcggtatcgt ctatcgataa 780

<210> 5
<211> 939
<212> DNA
<213> Saccharomyces cerevisiae

<400> 5
atgcctgcta ctttacatga ttctacgaaa atcctttctc taaatactgg agcccaaatc 60

cctcaaatag gtttaggtac gtggcagtcg aaagagaacg atgcttataa ggctgtttta 120

accgctttga aagatggcta ccgacacatt gatactgctg ctatttaccg taatgaagac 180

caagtcggtc aagccatcaa ggattcaggt gttcctcggg aagaaatctt tgttactaca 240

aagttatggt gtacacaaca ccacgaacct gaagtagcgc tggatcaatc actaaagagg 300

ttaggattgg actacgtaga cttatatttg atgcattggc ctgccagatt agatccagcc 360

tacatcaaaa atgaagacat cttgagtgtg ccaacaaaga aggatggttc tcgtgcagtg 420

gatatcacca attggaattt catcaaaacc tgggaattaa tgcaggaact accaaagact 480

ggtaaaacta aggccgttgg agtctccaac ttttctataa ataacctgaa agatctatta 540

gcatctcaag gtaataagct tacgccagct gctaaccaag tcgaaataca tccattacta 600

cctcaagacg aattgattaa tttttgtaaa agtaaaggca ttgtggttga agcttattct 660

ccgttaggta gtaccgatgc tccactattg aaggaaccgg ttatccttga aattgcgaag 720

aaaaataacg ttcaacccgg acacgttgtt attagctggc acgtccaaag aggttatgtt 780

gtcttgccaa aatctgtgaa tcccgatcga atcaaaacga acaggaaaat atttactttg 840

tctactgagg actttgaagc tatcaataac atatcgaagg aaaagggcga aaaaagggtt 900

gtacatccaa attggtctcc tttcgaagta ttcaagtaa 939

<210> 6
<211> 825
<212> DNA
<213> Hyoscyamus niger

<400> 6
atggccggag aatcagaagt ttacattaat ggcaacaatg gaggaattag atggagtctc 60

aaaggcacaa ctgcccttgt tactggtggc tctaaaggca ttgggtatgc agtagtggaa 120

gaactagcag gtcttggtgc aagagtatat acatgttcac gtaatgaaaa ggaactccaa 180

caatgccttg atatttggag aaatgaagga cttcaagttg aaggttctgt ttgtgattta 240

ttactgcgct ctgaacgtga caaacttatg cagactgttg cagatttatt taatggaaag 300

ctcaatattt tggtaaataa tgcaggtgtg gtgatacata aagaagctaa agatttcaca 360

aaagaagatt acgacatcgt attgggcact aattttgaag cagcttatca cttatgtcaa 420

cttgcttatc cctttttgaa ggcatctcaa aatggcaatg ttatttttct ttcttctata 480

gctggatttt cagcactgcc ttctgtttct ctttattctg cttccaaagc tgcaataaat 540

caaataacga agaacttggc atgtgaatgg gccaaggaca acattcgggt caattcagtt 600

gctccaggag tcattttaac cccactcatt gaaactgcaa ttaagaaaaa tcctcatcaa 660

aaagaagaaa tagacaattt tattgtcaag actccaatgg gccgggctgg aaagcccaat 720

gaagtgtctg cactaatagc ctttctttgc ttccctgctg cttcttatat tactggccaa 780

attatatggg ctgatggtgg attcacagct aatggtgggt tttaa 825

<210> 7
<211> 822
<212> DNA
<213> Datura stramonium

<400> 7
atggaagaat caaaagtgtc catgatgaat tgcaacaatg aaggaagatg gagtctcaaa 60

ggcaccacag cccttgttac tggtggctct aaaggcattg ggtatgcaat agtggaagaa 120

ttggcaggtc ttggagcaag agtatataca tgttcacgta atgaaaaaga actggacgaa 180

tgccttgaaa tttggagaga aaaaggactt aatgttgaag gttctgtttg tgacttatta 240

tcacgtactg aacgtgataa gcttatgcag actgttgcac atgtatttga tggaaagctc 300

aatattttgg tgaataatgc cggggtggtg atacataagg aagctaaaga tttcacagaa 360

aaagattaca acataattat gggaactaat tttgaagcag cttatcattt atctcaaatt 420

gcttatccat tattgaaggc ttctcaaaat gggaatgtta tttttctctc ttctattgct 480

ggattttcag cactgccttc tgtttctctt tactcagctt ccaaaggtgc aataaatcaa 540

atgacaaaga gtttggcttg tgaatgggct aaagacaaca ttcgggtcaa ttcagttgct 600

ccgggagtca ttttaacccc actggttgaa actgcaatta agaaaaatcc tcatcaaaaa 660

gaagaaatag acaattttat tgtcaagact cctatgggcc gggccggaaa gccccaagaa 720

gtttctgcac taatagcttt tctttgcttc cctgctgctt catatattac gggccagatc 780

atatgggctg acggtggatt cacagctaat ggtgggtttt aa 822

<210> 8
<211> 1014
<212> DNA
<213> Geobacillus stearothermophilus

<400> 8
atgaaagctg cagttgtgga acaatttaaa aagccgttac aagtgaaaga agtggaaaaa 60

cctaagatct catacgggga agtattagtg cgcatcaaag cgtgtggggt atgccataca 120

gacttgcatg ccgcacatgg cgactggcct gtaaagccta aactgcctct cattcctggc 180

catgaaggcg tcggtgtaat tgaagaagta ggtcctgggg taacacattt aaaagttgga 240

gatcgcgtag gtatcccttg gctttattcg gcgtgcggtc attgtgacta ttgcttaagc 300

ggacaagaaa cattatgcga acgtcaacaa aacgctggct attccgtcga tggtggttat 360

gctgaatatt gccgtgctgc agccgattat gtcgtaaaaa ttcctgataa cttatcgttt 420

gaagaagccg ctccaatctt ttgcgctggt gtaacaacat ataaagcgct caaagtaaca 480

ggcgcaaaac caggtgaatg ggtagccatt tacggtatcg gcgggcttgg acatgtcgca 540

gtccaatacg caaaggcgat ggggttaaac gtcgttgctg tcgatttagg tgatgaaaaa 600

cttgagcttg ctaaacaact tggtgcagat cttgtcgtca atccgaaaca tgatgatgca 660

gcacaatgga taaaagaaaa agtgggcggt gtgcatgcga ctgtcgtcac agctgtttca 720

aaagccgcgt tcgaatcagc ctacaaatcc attcgtcgcg gtggtgcttg cgtactcgtc 780

ggattaccgc cggaagaaat acctattcca attttcgata cagtattaaa tggagtaaaa 840

attattggtt ctatcgttgg tacgcgcaaa gacttacaag aggcacttca atttgcagca 900

gaaggaaaag taaaaacaat tgtcgaagtg caaccgcttg aaaacattaa cgacgtattc 960

gatcgtatgt taaaagggca aattaacggc cgcgtcgtgt taaaagtaga ttaa 1014

<210> 9
<211> 336
<212> PRT
<213> Candida parapsilosis

<400> 9
Met Ser Ile Pro Ser Ser Gln Tyr Gly Phe Val Phe Asn Lys Gln Ser
1               5                   10                  15

Gly Leu Asn Leu Arg Asn Asp Leu Pro Val His Lys Pro Lys Ala Gly
            20                  25                  30

Gln Leu Leu Leu Lys Val Asp Ala Val Gly Leu Cys His Ser Asp Leu
        35                  40                  45

His Val Ile Tyr Glu Gly Leu Asp Cys Gly Asp Asn Tyr Val Met Gly
    50                  55                  60

His Glu Ile Ala Gly Thr Val Ala Ala Val Gly Asp Asp Val Ile Asn
65                  70                  75                  80

Tyr Lys Val Gly Asp Arg Val Ala Cys Val Gly Pro Asn Gly Cys Gly
                85                  90                  95

Gly Cys Lys Tyr Cys Arg Gly Ala Ile Asp Asn Val Cys Lys Asn Ala
            100                 105                 110

Phe Gly Asp Trp Phe Gly Leu Gly Tyr Asp Gly Gly Tyr Gln Gln Tyr
        115                 120                 125

Leu Leu Val Thr Arg Pro Arg Asn Leu Ser Arg Ile Pro Asp Asn Val
    130                 135                 140

Ser Ala Asp Val Ala Ala Ala Ser Thr Asp Ala Val Leu Thr Pro Tyr
145                 150                 155                 160

His Ala Ile Lys Met Ala Gln Val Ser Pro Thr Ser Asn Ile Leu Leu
                165                 170                 175

Ile Gly Ala Gly Gly Leu Gly Gly Asn Ala Ile Gln Val Ala Lys Ala
            180                 185                 190

Phe Gly Ala Lys Val Thr Val Leu Asp Lys Lys Lys Glu Ala Arg Asp
        195                 200                 205

Gln Ala Lys Lys Leu Gly Ala Asp Ala Val Tyr Glu Thr Leu Pro Glu
    210                 215                 220

Ser Ile Ser Pro Gly Ser Phe Ser Ala Cys Phe Asp Phe Val Ser Val
225                 230                 235                 240

Gln Ala Thr Phe Asp Val Cys Gln Lys Tyr Val Glu Pro Lys Gly Val
                245                 250                 255

Ile Met Pro Val Gly Leu Gly Ala Pro Asn Leu Ser Phe Asn Leu Gly
            260                 265                 270

Asp Leu Ala Leu Arg Glu Ile Arg Ile Leu Gly Ser Phe Trp Gly Thr
        275                 280                 285

Thr Asn Asp Leu Asp Asp Val Leu Lys Leu Val Ser Glu Gly Lys Val
    290                 295                 300

Lys Pro Val Val Arg Ser Ala Lys Leu Lys Glu Leu Pro Glu Tyr Ile
305                 310                 315                 320

Glu Lys Leu Arg Asn Asn Ala Tyr Glu Gly Arg Val Val Phe Asn Pro
                325                 330                 335

<210>  10
<211>  348
<212>  PRT
<213>  Rhodococcus erythropolis

<400>  10
Met Lys Ala Ile Gln Tyr Thr Arg Ile Gly Ala Glu Pro Glu Leu Thr
1               5                   10                  15

Glu Ile Pro Lys Pro Glu Pro Gly Pro Gly Glu Val Leu Leu Glu Val
            20                  25                  30

Thr Ala Ala Gly Val Cys His Ser Asp Asp Phe Ile Met Ser Leu Pro

35                  40                  45

Glu Glu Gln Tyr Thr Tyr Gly Leu Pro Leu Thr Leu Gly His Glu Gly
    50                  55                  60

Ala Gly Lys Val Ala Ala Val Gly Glu Gly Val Glu Gly Leu Asp Ile
65                  70                  75                  80

Gly Thr Asn Val Val Val Tyr Gly Pro Trp Gly Cys Gly Asn Cys Trp
                85                  90                  95

His Cys Ser Gln Gly Leu Glu Asn Tyr Cys Ser Arg Ala Gln Glu Leu
            100                 105                 110

Gly Ile Asn Pro Pro Gly Leu Gly Ala Pro Gly Ala Leu Ala Glu Phe
        115                 120                 125

Met Ile Val Asp Ser Pro Arg His Leu Val Pro Ile Gly Asp Leu Asp
    130                 135                 140

Pro Val Lys Thr Val Pro Leu Thr Asp Ala Gly Leu Thr Pro Tyr His
145                 150                 155                 160

Ala Ile Lys Arg Ser Leu Pro Lys Leu Arg Gly Gly Ser Phe Ala Val
                165                 170                 175

Val Ile Gly Thr Gly Gly Leu Gly His Val Ala Ile Gln Leu Leu Arg
            180                 185                 190

His Leu Ser Ala Ala Thr Val Ile Ala Leu Asp Val Ser Ala Asp Lys
        195                 200                 205

Leu Glu Leu Ala Thr Lys Val Gly Ala His Glu Val Val Leu Ser Asp
    210                 215                 220

Lys Asp Ala Ala Glu Asn Val Arg Lys Ile Thr Gly Ser Gln Gly Ala
225                 230                 235                 240

Ala Leu Val Leu Asp Phe Val Gly Tyr Gln Pro Thr Ile Asp Thr Ala
                245                 250                 255

Met Ala Val Ala Gly Val Gly Ser Asp Val Thr Ile Val Gly Ile Gly
            260                 265                 270

Asp Gly Gln Ala His Ala Lys Val Gly Phe Phe Gln Ser Pro Tyr Glu
        275                 280                 285

Ala Ser Val Thr Val Pro Tyr Trp Gly Ala Arg Asn Glu Leu Ile Glu
    290                 295                 300

Leu Ile Asp Leu Ala His Ala Gly Ile Phe Asp Ile Ala Val Glu Thr
305                 310                 315                 320

Phe Ser Leu Asp Asn Gly Ala Glu Ala Tyr Arg Arg Leu Ala Ala Gly
                325                 330                 335

Thr Leu Ser Gly Arg Ala Val Val Val Pro Gly Leu
            340                 345

<210>  11
<211>  346
<212>  PRT
<213>  Streptomyces coelicolor

<400>  11
Met Lys Ala Leu Gln Tyr Arg Thr Ile Gly Ala Pro Pro Glu Val Val
1               5                   10                  15

Thr Val Pro Asp Pro Glu Pro Gly Pro Gly Gln Val Leu Leu Lys Val
            20                  25                  30

Thr Ala Ala Gly Val Cys His Ser Asp Ile Ala Val Met Ser Trp Pro
        35                  40                  45

Ala Glu Gly Phe Pro Tyr Glu Leu Pro Leu Thr Leu Gly His Glu Gly
    50                  55                  60

Val Gly Thr Val Ala Ala Leu Gly Ala Gly Val Thr Gly Leu Ala Glu
65                  70                  75                  80

Gly Asp Ala Val Ala Val Tyr Gly Pro Trp Gly Cys Gly Thr Cys Ala
                85                  90                  95

Lys Cys Ala Glu Gly Lys Glu Asn Tyr Cys Leu Arg Ala Asp Glu Leu
            100                 105                 110

Gly Ile Arg Pro Pro Gly Leu Gly Arg Pro Gly Ser Met Ala Glu Tyr
        115                 120                 125

Leu Leu Ile Asp Asp Pro Arg His Leu Val Pro Leu Asp Gly Leu Asp
    130                 135                 140

Pro Val Ala Ala Val Pro Leu Thr Asp Ala Gly Leu Thr Pro Tyr His
145                 150                 155                 160

Ala Ile Lys Arg Ser Leu Pro Lys Leu Val Pro Gly Ser Thr Ala Val
                165                 170                 175

Val Ile Gly Thr Gly Gly Leu Gly His Val Ala Ile Gln Leu Leu Arg
            180                 185                 190

Ala Leu Thr Ser Ala Arg Val Val Ala Leu Asp Val Ser Glu Glu Lys
        195                 200                 205

Leu Arg Leu Ala Arg Ala Val Gly Ala His Glu Ala Val Leu Ser Asp
    210                 215                 220

Ala Lys Ala Ala Asp Ala Val Arg Glu Ile Thr Gly Gly Leu Gly Ala
225                 230                 235                 240

Glu Ala Val Phe Asp Phe Val Gly Val Ala Pro Thr Val Gln Thr Ala
                245                 250                 255

Gly Ala Val Ala Ala Val Glu Gly Asp Val Thr Leu Val Gly Ile Gly
            260                 265                 270

Gly Gly Ser Leu Pro Val Gly Phe Gly Met Leu Pro Phe Glu Val Ser
        275                 280                 285

Val Asn Ala Pro Tyr Trp Gly Ser Arg Ser Glu Leu Thr Glu Val Leu
    290                 295                 300

Asn Leu Ala Arg Ser Gly Ala Val Ser Val His Thr Glu Thr Tyr Ser
305                 310                 315                 320

Leu Asp Asp Ala Pro Leu Ala Tyr Glu Arg Leu His Glu Gly Arg Val
                325                 330                 335

Asn Gly Arg Ala Val Ile Leu Pro His Gly
            340                 345

<210>  12
<211>  259
<212>  PRT
<213>  Zoogloea ramigera

<400>  12
Met Ser Leu Asn Gly Lys Val Ile Leu Val Thr Gly Ala Gly Gln Gly
1               5                   10                  15

Ile Gly Arg Gly Ile Ala Leu Arg Leu Ala Lys Glu Gly Ala Asp Leu
            20                  25                  30

Ala Leu Ala Asp Val Lys Ala Asp Lys Leu Asp Ser Val Arg Lys Glu
        35                  40                  45

Val Glu Ala Leu Gly Arg Lys Ala Thr Thr Val Val Ala Asp Val Ser
    50                  55                  60

Lys Arg Asp Glu Val Tyr Ala Ala Ile Asp His Ala Glu Lys Gln Leu
65                  70                  75                  80

Gly Gly Phe Asp Val Met Val Asn Asn Ala Gly Ile Ala Gln Val Lys
                85                  90                  95

Pro Ile Ala Asp Val Thr Pro Glu Asp Met Asp Leu Ile Phe Arg Ile
            100                 105                 110

Asn Val Asp Gly Val Leu Trp Gly Ile Gln Ala Ala Ser Gln Lys Phe
        115                 120                 125

Lys Asp Arg Gly Gln Lys Gly Lys Ile Ile Asn Ala Cys Ser Ile Ala
    130                 135                 140

Gly His Asp Gly Phe Ala Met Leu Gly Val Tyr Ser Ala Thr Lys Phe
145                 150                 155                 160

Ala Val Arg Ala Leu Thr Gln Ala Ala Ala Lys Glu Tyr Ala Ser Ala
                165                 170                 175

Gly Ile Thr Val Asn Ala Tyr Cys Pro Gly Ile Val Gly Thr Asp Met
            180                 185                 190

Trp Val Glu Ile Asp Glu Arg Phe Ser Glu Ile Thr Gly Thr Pro Lys
        195                 200                 205

Gly Glu Thr Tyr Lys Lys Tyr Val Glu Gly Ile Ala Leu Gly Arg Ala
    210                 215                 220

Gln Thr Pro Glu Asp Val Ala Ala Leu Val Ala Phe Leu Ala Gly Ala
225                 230                 235                 240

Asp Ser Asp Tyr Ile Thr Gly Gln Ser Ile Leu Thr Asp Gly Gly Ile
                245                 250                 255

Val Tyr Arg


<210>  13
<211>  312
<212>  PRT
<213>  Saccharomyces cerevisiae

<400>  13
Met Pro Ala Thr Leu His Asp Ser Thr Lys Ile Leu Ser Leu Asn Thr
1               5                   10                  15

Gly Ala Gln Ile Pro Gln Ile Gly Leu Gly Thr Trp Gln Ser Lys Glu
            20                  25                  30

Asn Asp Ala Tyr Lys Ala Val Leu Thr Ala Leu Lys Asp Gly Tyr Arg
        35                  40                  45

His Ile Asp Thr Ala Ala Ile Tyr Arg Asn Glu Asp Gln Val Gly Gln

50                  55                  60

Ala Ile Lys Asp Ser Gly Val Pro Arg Glu Glu Ile Phe Val Thr Thr
65                  70                  75                  80

Lys Leu Trp Cys Thr Gln His His Glu Pro Glu Val Ala Leu Asp Gln
                85                  90                  95

Ser Leu Lys Arg Leu Gly Leu Asp Tyr Val Asp Leu Tyr Leu Met His
            100                 105                 110

Trp Pro Ala Arg Leu Asp Pro Ala Tyr Ile Lys Asn Glu Asp Ile Leu
        115                 120                 125

Ser Val Pro Thr Lys Lys Asp Gly Ser Arg Ala Val Asp Ile Thr Asn
    130                 135                 140

Trp Asn Phe Ile Lys Thr Trp Glu Leu Met Gln Glu Leu Pro Lys Thr
145                 150                 155                 160

Gly Lys Thr Lys Ala Val Gly Val Ser Asn Phe Ser Ile Asn Asn Leu
                165                 170                 175

Lys Asp Leu Leu Ala Ser Gln Gly Asn Lys Leu Thr Pro Ala Ala Asn
            180                 185                 190

Gln Val Glu Ile His Pro Leu Leu Pro Gln Asp Glu Leu Ile Asn Phe
        195                 200                 205

Cys Lys Ser Lys Gly Ile Val Val Glu Ala Tyr Ser Pro Leu Gly Ser
    210                 215                 220

Thr Asp Ala Pro Leu Leu Lys Glu Pro Val Ile Leu Glu Ile Ala Lys
225                 230                 235                 240

Lys Asn Asn Val Gln Pro Gly His Val Val Ile Ser Trp His Val Gln
                245                 250                 255

Arg Gly Tyr Val Val Leu Pro Lys Ser Val Asn Pro Asp Arg Ile Lys
            260                 265                 270

Thr Asn Arg Lys Ile Phe Thr Leu Ser Thr Glu Asp Phe Glu Ala Ile
        275                 280                 285

Asn Asn Ile Ser Lys Glu Lys Gly Glu Lys Arg Val Val His Pro Asn
    290                 295                 300

Trp Ser Pro Phe Glu Val Phe Lys
305                 310


<210>  14
<211>  274
<212>  PRT
<213>  Hyoscyamus niger

<400>  14
Met Ala Gly Glu Ser Glu Val Tyr Ile Asn Gly Asn Asn Gly Gly Ile
1               5                   10                  15

Arg Trp Ser Leu Lys Gly Thr Thr Ala Leu Val Thr Gly Gly Ser Lys
            20                  25                  30

Gly Ile Gly Tyr Ala Val Val Glu Glu Leu Ala Gly Leu Gly Ala Arg
        35                  40                  45

Val Tyr Thr Cys Ser Arg Asn Glu Lys Glu Leu Gln Gln Cys Leu Asp
    50                  55                  60

Ile Trp Arg Asn Glu Gly Leu Gln Val Glu Gly Ser Val Cys Asp Leu
65                  70                  75                  80

Leu Leu Arg Ser Glu Arg Asp Lys Leu Met Gln Thr Val Ala Asp Leu
                85                  90                  95

Phe Asn Gly Lys Leu Asn Ile Leu Val Asn Asn Ala Gly Val Val Ile
            100                 105                 110

His Lys Glu Ala Lys Asp Phe Thr Lys Glu Asp Tyr Asp Ile Val Leu
        115                 120                 125

Gly Thr Asn Phe Glu Ala Ala Tyr His Leu Cys Gln Leu Ala Tyr Pro
    130                 135                 140

Phe Leu Lys Ala Ser Gln Asn Gly Asn Val Ile Phe Leu Ser Ser Ile
145                 150                 155                 160

Ala Gly Phe Ser Ala Leu Pro Ser Val Ser Leu Tyr Ser Ala Ser Lys
                165                 170                 175

Ala Ala Ile Asn Gln Ile Thr Lys Asn Leu Ala Cys Glu Trp Ala Lys
            180                 185                 190

Asp Asn Ile Arg Val Asn Ser Val Ala Pro Gly Val Ile Leu Thr Pro
        195                 200                 205

Leu Ile Glu Thr Ala Ile Lys Lys Asn Pro His Gln Lys Glu Glu Ile
    210                 215                 220

Asp Asn Phe Ile Val Lys Thr Pro Met Gly Arg Ala Gly Lys Pro Asn
225                 230                 235                 240

Glu Val Ser Ala Leu Ile Ala Phe Leu Cys Phe Pro Ala Ala Ser Tyr
                245                 250                 255

Ile Thr Gly Gln Ile Ile Trp Ala Asp Gly Gly Phe Thr Ala Asn Gly
            260                 265                 270

Gly Phe

<210>  15
<211>  273
<212>  PRT
<213>  Datura stramonium

<400>  15
Met Glu Glu Ser Lys Val Ser Met Met Asn Cys Asn Asn Glu Gly Arg
1               5                   10                  15

Trp Ser Leu Lys Gly Thr Thr Ala Leu Val Thr Gly Gly Ser Lys Gly
            20                  25                  30

Ile Gly Tyr Ala Ile Val Glu Glu Leu Ala Gly Leu Gly Ala Arg Val
        35                  40                  45

Tyr Thr Cys Ser Arg Asn Glu Lys Glu Leu Asp Glu Cys Leu Glu Ile
    50                  55                  60

Trp Arg Glu Lys Gly Leu Asn Val Glu Gly Ser Val Cys Asp Leu Leu
65                  70                  75                  80

Ser Arg Thr Glu Arg Asp Lys Leu Met Gln Thr Val Ala His Val Phe
                85                  90                  95

Asp Gly Lys Leu Asn Ile Leu Val Asn Asn Ala Gly Val Val Ile His
            100                 105                 110

Lys Glu Ala Lys Asp Phe Thr Glu Lys Asp Tyr Asn Ile Ile Met Gly
        115                 120                 125

Thr Asn Phe Glu Ala Ala Tyr His Leu Ser Gln Ile Ala Tyr Pro Leu
    130                 135                 140

Leu Lys Ala Ser Gln Asn Gly Asn Val Ile Phe Leu Ser Ser Ile Ala
145                 150                 155                 160

Gly Phe Ser Ala Leu Pro Ser Val Ser Leu Tyr Ser Ala Ser Lys Gly
                165                 170                 175

Ala Ile Asn Gln Met Thr Lys Ser Leu Ala Cys Glu Trp Ala Lys Asp
            180                 185                 190

Asn Ile Arg Val Asn Ser Val Ala Pro Gly Val Ile Leu Thr Pro Leu
        195                 200                 205

Val Glu Thr Ala Ile Lys Lys Asn Pro His Gln Lys Glu Glu Ile Asp
    210                 215                 220

Asn Phe Ile Val Lys Thr Pro Met Gly Arg Ala Gly Lys Pro Gln Glu
225                 230                 235                 240

Val Ser Ala Leu Ile Ala Phe Leu Cys Phe Pro Ala Ala Ser Tyr Ile
                245                 250                 255

Thr Gly Gln Ile Ile Trp Ala Asp Gly Gly Phe Thr Ala Asn Gly Gly
            260                 265                 270

Phe

<210>  16
<211>  337
<212>  PRT
<213>  Geobacillus stearothermophilus

<400>  16
Met Lys Ala Ala Val Val Glu Gln Phe Lys Lys Pro Leu Gln Val Lys
1               5                   10                  15

Glu Val Glu Lys Pro Lys Ile Ser Tyr Gly Glu Val Leu Val Arg Ile
            20                  25                  30

Lys Ala Cys Gly Val Cys His Thr Asp Leu His Ala Ala His Gly Asp
        35                  40                  45

Trp Pro Val Lys Pro Lys Leu Pro Leu Ile Pro Gly His Glu Gly Val
    50                  55                  60

Gly Val Ile Glu Glu Val Gly Pro Gly Val Thr His Leu Lys Val Gly
65                  70                  75                  80

Asp Arg Val Gly Ile Pro Trp Leu Tyr Ser Ala Cys Gly His Cys Asp
                85                  90                  95

Tyr Cys Leu Ser Gly Gln Glu Thr Leu Cys Glu Arg Gln Gln Asn Ala
            100                 105                 110

Gly Tyr Ser Val Asp Gly Gly Tyr Ala Glu Tyr Cys Arg Ala Ala Ala
        115                 120                 125

Asp Tyr Val Val Lys Ile Pro Asp Asn Leu Ser Phe Glu Glu Ala Ala
    130                 135                 140

Pro Ile Phe Cys Ala Gly Val Thr Thr Tyr Lys Ala Leu Lys Val Thr

145                 150                 155                 160

Gly Ala Lys Pro Gly Glu Trp Val Ala Ile Tyr Gly Ile Gly Gly Leu
                165                 170                 175

Gly His Val Ala Val Gln Tyr Ala Lys Ala Met Gly Leu Asn Val Val
            180                 185                 190

Ala Val Asp Leu Gly Asp Glu Lys Leu Glu Leu Ala Lys Gln Leu Gly
        195                 200                 205

Ala Asp Leu Val Val Asn Pro Lys His Asp Asp Ala Ala Gln Trp Ile
    210                 215                 220

Lys Glu Lys Val Gly Gly Val His Ala Thr Val Val Thr Ala Val Ser
225                 230                 235                 240

Lys Ala Ala Phe Glu Ser Ala Tyr Lys Ser Ile Arg Arg Gly Gly Ala
                245                 250                 255

Cys Val Leu Val Gly Leu Pro Pro Glu Glu Ile Pro Ile Pro Ile Phe
            260                 265                 270

Asp Thr Val Leu Asn Gly Val Lys Ile Ile Gly Ser Ile Val Gly Thr
        275                 280                 285

Arg Lys Asp Leu Gln Glu Ala Leu Gln Phe Ala Ala Glu Gly Lys Val
    290                 295                 300

Lys Thr Ile Val Glu Val Gln Pro Leu Glu Asn Ile Asn Asp Val Phe
305                 310                 315                 320

Asp Arg Met Leu Lys Gly Gln Ile Asn Gly Arg Val Val Leu Lys Val
                325                 330                 335

Asp


<210>  17
<211>  765
<212>  DNA
<213>  Pichia finlandica

<400>  17
atgtcttata acttccataa caaggttgca gttgttactg gagctctatc aggaatcggc      60

ttaagcgtcg caaaaaagtt ccttcagctc ggcgccaaag taacgatctc tgatgtcagt     120

ggagagaaaa aatatcacga gactgttgtt gctctgaaag cccaaaatct caacactgac     180

aacctccatt atgtacaggc agattccagc aaagaagaag ataacaagaa attgatttcg     240

gaaactctgg caacctttgg gggcctggat attgtttgtg ctaatgcagg aattggaaag     300

ttcgctccca cccatgaaac acccttcgac gtatggaaga aggtgattgc tgtgaatttg     360

aatggagtat tcttactgga taagctagcc atcaattact ggctagagaa aagcaaaccc     420

ggcgtaattg tcaacatggg atcagtccac tcttttgtag cagctcctgg ccttgcgcat     480

tatggagctg caaaaggcgg tgtcaaactg ttaacacaaa cattggctct agagtacgca     540

tctcatggta ttagagtaaa ttctgtcaat ccggggtaca tttcgactcc tttgatagat     600

gaggttccga aagagcggtt ggataaactt gtaagcttgc accctattgg gagactaggt     660

cgtccagagg aagttgctga tgcagtcgca tttctgtgtt cccaggaggc cactttcatc     720

aacggcgttt ctttgccggt tgacgggggg tacacagccc agtaa 765

<210> 18
<211> 254
<212> PRT
<213> Pichia finlandica

<400> 18
Met Ser Tyr Asn Phe His Asn Lys Val Ala Val Val Thr Gly Ala Leu
1               5                   10                  15

Ser Gly Ile Gly Leu Ser Val Ala Lys Lys Phe Leu Gln Leu Gly Ala
            20                  25                  30

Lys Val Thr Ile Ser Asp Val Ser Gly Glu Lys Lys Tyr His Glu Thr
        35                  40                  45

Val Val Ala Leu Lys Ala Gln Asn Leu Asn Thr Asp Asn Leu His Tyr
    50                  55                  60

Val Gln Ala Asp Ser Ser Lys Glu Glu Asp Asn Lys Lys Leu Ile Ser
65                  70                  75                  80

Glu Thr Leu Ala Thr Phe Gly Gly Leu Asp Ile Val Cys Ala Asn Ala
                85                  90                  95

Gly Ile Gly Lys Phe Ala Pro Thr His Glu Thr Pro Phe Asp Val Trp
            100                 105                 110

Lys Lys Val Ile Ala Val Asn Leu Asn Gly Val Phe Leu Leu Asp Lys
        115                 120                 125

Leu Ala Ile Asn Tyr Trp Leu Glu Lys Ser Lys Pro Gly Val Ile Val
    130                 135                 140

Asn Met Gly Ser Val His Ser Phe Val Ala Ala Pro Gly Leu Ala His
145                 150                 155                 160

Tyr Gly Ala Ala Lys Gly Gly Val Lys Leu Leu Thr Gln Thr Leu Ala
                165                 170                 175

Leu Glu Tyr Ala Ser His Gly Ile Arg Val Asn Ser Val Asn Pro Gly
            180                 185                 190

Tyr Ile Ser Thr Pro Leu Ile Asp Glu Val Pro Lys Glu Arg Leu Asp
        195                 200                 205

Lys Leu Val Ser Leu His Pro Ile Gly Arg Leu Gly Arg Pro Glu Glu
    210                 215                 220

Val Ala Asp Ala Val Ala Phe Leu Cys Ser Gln Glu Ala Thr Phe Ile
225                 230                 235                 240

Asn Gly Val Ser Leu Pro Val Asp Gly Gly Tyr Thr Ala Gln
                245                 250

<210> 19
<211> 34
<212> DNA
<213> Artificial

<220>
<223> An artificially synthesized primer sequence

<400> 19
gtggaattct ataatgtcaa ttccatcaag ccag 34

<210>  20
<211>  43
<212>  DNA
<213>  Artificial

<220>
<223>  An artificially synthesized primer sequence

<400>  20
ctgaagctta ttatggatta aaaacaacac gaccttcata agc                43

<210>  21
<211>  34
<212>  DNA
<213>  Artificial

<220>
<223>  An artificially synthesized primer sequence

<400>  21
cacgaattct atcatgaaag caatccagta cacg                          34

<210>  22
<211>  35
<212>  DNA
<213>  Artificial

<220>
<223>  An artificially synthesized primer sequence

<400>  22
tcgaagcttc tagattaaag accagggacc acaac                         35

<210>  23
<211>  37
<212>  DNA
<213>  Artificial

<220>
<223>  An artificially synthesized primer sequence

<400>  23
gagccatggc acctgctact ttacatgatt ctacgaa                       37

<210>  24
<211>  42
<212>  DNA
<213>  Artificial

<220>
<223>  An artificially synthesized primer sequence

<400>  24
gagcttaagt ctagattatt tgaatacttc gaaaggagac ca                 42

<210>  25
<211>  31
<212>  DNA
<213>  Artificial

<220>
<223>  An artificially synthesized primer sequence

<400>  25
gaggaattca atcatgaaag ctgcagttgt g                                    31

<210>  26
<211>  37
<212>  DNA
<213>  Artificial

<220>
<223>  An artificially synthesized primer sequence

<400>  26
gtcaagcttc tagattaatc tacttttaac acgacgc                              37

<210>  27
<211>  39
<212>  DNA
<213>  Artificial

<220>
<223>  An artificially synthesized primer sequence

<400>  27
gtcgaattca tacatgtcta tcccagaaac tcaaaaagg                            39

<210>  28
<211>  47
<212>  DNA
<213>  Artificial

<220>
<223>  An artificially synthesized primer sequence

<400>  28
ctgcttaagt ctagattatt tagaagtgtc aacaacgtaa cgaccaa                   47

<210>  29
<211>  1047
<212>  DNA
<213>  Saccharomyces cerevisiae

<400>  29
atgtctatcc cagaaactca aaaaggtgtt atcttctacg aatcccacgg taaattggaa      60

cacaaggata ttccagttcc aaagccaaag gccaacgaat tgttgatcaa cgttaagtac     120

tctggtgtct gtcacaccga cttgcacgct tggcacggtg actggccatt gccagttaag     180

ctaccattag tcggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca acaatacgct accgctgacg ctgttcaagc cgctcacatt     420

cctcaaggta ccgacttggc ccaagtcgcc cccatcttgt gtgctggtat caccgtctac     480

aaggctttga agtctgctaa cttgatggcc ggtcattggg ttgccatttc cggtgctgcc     540

ggtggtctag gttctttggc tgttcaatac gccaaggcta tgggttacag agtcttgggt     600

attgacggtg gtgaaggtaa ggaagaatta ttcagatcca tcggtggtga agtcttcatt     660

gacttcacta aggaaaagga cattgtcggt gctgttctaa aggccactga cggtggtgct     720

cacggtgtca tcaacgtttc cgtttccgaa gccgctattg aagcttctac cagatacgtt 780

agagctaacg gtaccaccgt tttggtcggt atgccagctg gtgccaagtg ttgttctgat 840

gtcttcaacc aagtcgtcaa gtccatctct attgttggtt cttacgtcgg taacagagcc 900

gacaccagag aagctttgga cttcttcgcc agaggtttgg tcaagtctcc aatcaaggtt 960

gtcggcttgt ctaccttgcc agaaatttac gaaaagatgg aaaagggtca aatcgttggt 1020

agatacgttg ttgacacttc taaataa 1047

<210> 30
<211> 348
<212> PRT
<213> Saccharomyces cerevisiae

<400> 30
Met Ser Ile Pro Glu Thr Gln Lys Gly Val Ile Phe Tyr Glu Ser His
1               5                   10                  15

Gly Lys Leu Glu His Lys Asp Ile Pro Val Pro Lys Pro Lys Ala Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Val Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly
                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Gln
        115                 120                 125

Tyr Ala Thr Ala Asp Ala Val Gln Ala Ala His Ile Pro Gln Gly Thr
    130                 135                 140

Asp Leu Ala Gln Val Ala Pro Ile Leu Cys Ala Gly Ile Thr Val Tyr
145                 150                 155                 160

Lys Ala Leu Lys Ser Ala Asn Leu Met Ala Gly His Trp Val Ala Ile
                165                 170                 175

Ser Gly Ala Ala Gly Gly Leu Gly Ser Leu Ala Val Gln Tyr Ala Lys
            180                 185                 190

Ala Met Gly Tyr Arg Val Leu Gly Ile Asp Gly Gly Glu Gly Lys Glu
        195                 200                 205

Glu Leu Phe Arg Ser Ile Gly Gly Glu Val Phe Ile Asp Phe Thr Lys
    210                 215                 220

Glu Lys Asp Ile Val Gly Ala Val Leu Lys Ala Thr Asp Gly Gly Ala
225                 230                 235                 240

His Gly Val Ile Asn Val Ser Val Ser Glu Ala Ala Ile Glu Ala Ser
                245                 250                 255

Thr Arg Tyr Val Arg Ala Asn Gly Thr Thr Val Leu Val Gly Met Pro

260                 265                 270

Ala Gly Ala Lys Cys Cys Ser Asp Val Phe Asn Gln Val Val Lys Ser
        275                 280                 285

Ile Ser Ile Val Gly Ser Tyr Val Gly Asn Arg Ala Asp Thr Arg Glu
    290                 295                 300

Ala Leu Asp Phe Phe Ala Arg Gly Leu Val Lys Ser Pro Ile Lys Val
305                 310                 315                 320

Val Gly Leu Ser Thr Leu Pro Glu Ile Tyr Glu Lys Met Glu Lys Gly
                325                 330                 335

Gln Ile Val Gly Arg Tyr Val Val Asp Thr Ser Lys
            340                 345

<210>  31
<211>  39
<212>  DNA
<213>  Artificial

<220>
<223>  An artificially synthesized primer sequence

<400>  31
gtcgaattca tacatgtcta ttccagaaac tcaaaaagc                              39

<210>  32
<211>  47
<212>  DNA
<213>  Artificial

<220>
<223>  An artificially synthesized primer sequence

<400>  32
gcacttaagt ctagattatt tagaagtgtc aacaacgtaa cgaccag                     47

<210>  33
<211>  1047
<212>  DNA
<213>  Saccharomyces cerevisiae

<400>  33
atgtctattc cagaaactca aaaagccatt atcttctacg aatccaacgg caagttggag      60

cataaggata tcccagttcc aaagccaaag cccaacgaat tgttaatcaa cgtcaagtac     120

tctggtgtct gccacaccga tttgcacgct tggcatggtg actggccatt gccaactaag     180

ttaccattag ttggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca agaatacgct accgctgacg ctgttcaagc cgctcacatt     420

cctcaaggta ctgacttggc tgaagtcgcg ccaatcttgt gtgctggtat caccgtatac     480

aaggctttga agtctgccaa cttgagagca ggccactggg cggccatttc tggtgctgct     540

ggtggtctag gttctttggc tgttcaatat gctaaggcga tgggttacag agtcttaggt     600

attgatggtg gtccaggaaa ggaagaattg tttacctcgc tcggtggtga agtattcatc     660

gacttcacca aagagaagga cattgttagc gcagtcgtta aggctaccaa cggcggtgcc 720

cacggtatca tcaatgtttc cgtttccgaa gccgctatcg aagcttctac cagatactgt 780

agggcgaacg gtactgttgt cttggttggt ttgccagccg gtgcaaagtg ctcctctgat 840

gtcttcaacc acgttgtcaa gtctatctcc attgtcggct cttacgtggg gaacagagct 900

gataccagag aagccttaga tttctttgcc agaggtctag tcaagtctcc aataaaggta 960

gttggcttat ccagtttacc agaaatttac gaaaagatgg agaagggcca aattgctggt 1020

cgttacgttg ttgacacttc taaataa 1047

<210> 34
<211> 348
<212> PRT
<213> Saccharomyces cerevisiae

<400> 34
Met Ser Ile Pro Glu Thr Gln Lys Ala Ile Ile Phe Tyr Glu Ser Asn
1               5                   10                  15

Gly Lys Leu Glu His Lys Asp Ile Pro Val Pro Lys Pro Lys Pro Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Thr Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly
                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Glu
        115                 120                 125

Tyr Ala Thr Ala Asp Ala Val Gln Ala Ala His Ile Pro Gln Gly Thr
    130                 135                 140

Asp Leu Ala Glu Val Ala Pro Ile Leu Cys Ala Gly Ile Thr Val Tyr
145                 150                 155                 160

Lys Ala Leu Lys Ser Ala Asn Leu Arg Ala Gly His Trp Ala Ala Ile
                165                 170                 175

Ser Gly Ala Ala Gly Gly Leu Gly Ser Leu Ala Val Gln Tyr Ala Lys
            180                 185                 190

Ala Met Gly Tyr Arg Val Leu Gly Ile Asp Gly Gly Pro Gly Lys Glu
        195                 200                 205

Glu Leu Phe Thr Ser Leu Gly Gly Glu Val Phe Ile Asp Phe Thr Lys
    210                 215                 220

Glu Lys Asp Ile Val Ser Ala Val Val Lys Ala Thr Asn Gly Gly Ala
225                 230                 235                 240

His Gly Ile Ile Asn Val Ser Val Ser Glu Ala Ala Ile Glu Ala Ser
                245                 250                 255

Thr Arg Tyr Cys Arg Ala Asn Gly Thr Val Val Leu Val Gly Leu Pro
            260                 265                 270

Ala Gly Ala Lys Cys Ser Ser Asp Val Phe Asn His Val Val Lys Ser
        275                 280                 285

Ile Ser Ile Val Gly Ser Tyr Val Gly Asn Arg Ala Asp Thr Arg Glu
    290                 295                 300

Ala Leu Asp Phe Phe Ala Arg Gly Leu Val Lys Ser Pro Ile Lys Val
305                 310                 315                 320

Val Gly Leu Ser Ser Leu Pro Glu Ile Tyr Glu Lys Met Glu Lys Gly
                325                 330                 335

Gln Ile Ala Gly Arg Tyr Val Val Asp Thr Ser Lys
            340                 345

<210>  35
<211>  36
<212>  DNA
<213>  Artificial

<220>
<223>  An artificially synthesized primer sequence

<400>  35
gagtcatgag ttcactggtt actcttaata acggtc                                36

<210>  36
<211>  43
<212>  DNA
<213>  Artificial

<220>
<223>  An artificially synthesized primer sequence

<400>  36
gacgaattcc tctagattat gcaaaagtgg ggaatttacc atc                        43

<210>  37
<211>  984
<212>  DNA
<213>  Saccharomyces cerevisiae

<400>  37
atgagttcac tggttactct taataacggt ctgaaaatgc ccctagtcgg cttagggtgc      60

tggaaaattg acaaaaaagt ctgtgcgaat caaatttatg aagctatcaa attaggctac     120

cgtttattcg atggtgcttg cgactacggc aacgaaaagg aagttggtga aggtatcagg     180

aaagccatct ccgaaggtct tgtttctaga aaggatatat ttgttgtttc aaagttatgg     240

aacaattttc accatcctga tcatgtaaaa ttagctttaa agaagacctt aagcgatatg     300

ggacttgatt atttagacct gtattatatt cacttcccaa tcgccttcaa atatgttcca     360

tttgaagaga aataccctcc aggattctat acgggcgcag atgacgagaa gaaaggtcac     420

atcaccgaag cacatgtacc aatcatagat acgtaccggg ctctggaaga atgtgttgat     480

gaaggcttga ttaagtctat tggtgtttcc aactttcagg gaagcttgat tcaagattta     540

ttacgtggtt gtagaatcaa gcccgtggct ttgcaaattg aacaccatcc ttatttgact     600

caagaacacc tagttgagtt ttgtaaatta cacgatatcc aagtagttgc ttactcctcc 660

ttcggtcctc aatcattcat tgagatggac ttacagttgg caaaaaccac gccaactctg 720

ttcgagaatg atgtaatcaa gaaggtctca caaaaccatc caggcagtac cacttcccaa 780

gtattgctta gatgggcaac tcagagaggc attgccgtca ttccaaaatc ttccaagaag 840

gaaaggttac ttggcaacct agaaatcgaa aaaaagttca ctttaacgga gcaagaattg 900

aaggatattt ctgcactaaa tgccaacatc agatttaatg atccatggac ctggttggat 960

ggtaaattcc ccacttttgc ataa 984

<210> 38
<211> 327
<212> PRT
<213> Saccharomyces cerevisiae

<400> 38
Met Ser Ser Leu Val Thr Leu Asn Asn Gly Leu Lys Met Pro Leu Val
1               5                   10                  15

Gly Leu Gly Cys Trp Lys Ile Asp Lys Lys Val Cys Ala Asn Gln Ile
            20                  25                  30

Tyr Glu Ala Ile Lys Leu Gly Tyr Arg Leu Phe Asp Gly Ala Cys Asp
        35                  40                  45

Tyr Gly Asn Glu Lys Glu Val Gly Glu Gly Ile Arg Lys Ala Ile Ser
    50                  55                  60

Glu Gly Leu Val Ser Arg Lys Asp Ile Phe Val Val Ser Lys Leu Trp
65                  70                  75                  80

Asn Asn Phe His His Pro Asp His Val Lys Leu Ala Leu Lys Lys Thr
                85                  90                  95

Leu Ser Asp Met Gly Leu Asp Tyr Leu Asp Leu Tyr Tyr Ile His Phe
            100                 105                 110

Pro Ile Ala Phe Lys Tyr Val Pro Phe Glu Glu Lys Tyr Pro Pro Gly
        115                 120                 125

Phe Tyr Thr Gly Ala Asp Asp Glu Lys Lys Gly His Ile Thr Glu Ala
    130                 135                 140

His Val Pro Ile Ile Asp Thr Tyr Arg Ala Leu Glu Glu Cys Val Asp
145                 150                 155                 160

Glu Gly Leu Ile Lys Ser Ile Gly Val Ser Asn Phe Gln Gly Ser Leu
                165                 170                 175

Ile Gln Asp Leu Leu Arg Gly Cys Arg Ile Lys Pro Val Ala Leu Gln
            180                 185                 190

Ile Glu His His Pro Tyr Leu Thr Gln Glu His Leu Val Glu Phe Cys
        195                 200                 205

Lys Leu His Asp Ile Gln Val Val Ala Tyr Ser Ser Phe Gly Pro Gln
    210                 215                 220

Ser Phe Ile Glu Met Asp Leu Gln Leu Ala Lys Thr Thr Pro Thr Leu
225                 230                 235                 240

Phe Glu Asn Asp Val Ile Lys Lys Val Ser Gln Asn His Pro Gly Ser
                245                 250                 255

Thr Thr Ser Gln Val Leu Leu Arg Trp Ala Thr Gln Arg Gly Ile Ala
            260                 265                 270

Val Ile Pro Lys Ser Ser Lys Lys Glu Arg Leu Leu Gly Asn Leu Glu
        275                 280                 285

Ile Glu Lys Lys Phe Thr Leu Thr Glu Gln Glu Leu Lys Asp Ile Ser
    290                 295                 300

Ala Leu Asn Ala Asn Ile Arg Phe Asn Asp Pro Trp Thr Trp Leu Asp
305                 310                 315                 320

Gly Lys Phe Pro Thr Phe Ala
                325

## Description

### Technical Field

The present invention relates to methods for producing optically active fluorine-containing compounds that are useful as optically active raw materials for various types of pharmaceutical products, liquid crystalline materials, and such.

### Background Art

The methods described below in <1> to <4> are known as methods for producing (S)-1,1,1-trifluoro-2-propanol represented by formula (2) 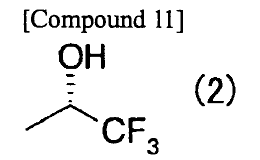 and (R)-1,1,1-trifluoro-2-propanol represented by formula (3). 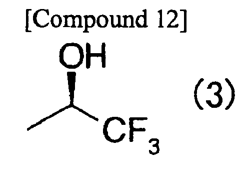
<1> the method for asymmetrically reducing 1,1,1-trifluoroacetone represented by formula (1) 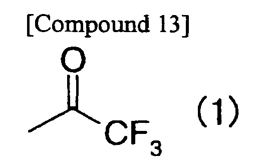 using baker's yeast (Non-Patent Document 1);
<2> the method for asymmetrically reducing 1,1,1-trifluoroacetone represented by formula (1) using DIP-C1, which is a chiral borane reducing agent (Non-Patent Document 2);
<3> the method for obtaining an optically active alcohol by asymmetrically reducing 1,1,1-trifluoro-3-bromoacetone using DIP-C1, which is a chiral borane reducing agent, then using a base to generate an optically active epoxide, which is then subjected to ring-opening using a hydride (Non-Patent Document 3); and
<4> the method for obtaining an optically active alcohol by carrying out a kinetic optical resolution by performing a hydrolysis reaction using lipase on esters of a racemic mixture of the alcohols represented by formulas (2) and (3) (Non-Patent Document 4).

However, the methods of <1>, <2>, and <4> all have low optical purity. In the method of <1>, the substrate concentration is very low being 0.3%, but yet uses baker's yeast as a catalyst at 18% in water, which is the solvent. It also uses the auxiliary material, glucose, at 21% which is a large amount compared to the substrate. Thus, the method of <1> has very poor efficiency. The methods of <2> and <3> require expensive reducing agents. The method of <3> involves complicated steps. The method of <4> gives a theoretical yield less than 50%. Therefore, all of the methods carry issues as industrial production methods.

Information on prior art documents relating to the invention of this application is shown below.
[Non-Patent Document 1] Synthesis, 897-899 (1983).
[Non-Patent Document 2] Tetrahedron, 49 (9), 1725-1738 (1993).
[Non-Patent Document 3] J. Org. Chem., 60 (1), 41-46 (1995).
[Non-Patent Document 4] Chem. Lett., 855-856 (1996).

### Disclosure of the Invention

### [Problems to be Solved by the Invention]

The present invention was achieved in view of the above-mentioned problems. An objective of the present invention is to provide novel methods for producing optically active alcohols represented by formulas (2) and (3). More specifically, an objective is to provide methods for producing optically active alcohols represented by formulas (2) and (3) at a good yield, as well as high purity.

### [Means for Solving the Problems]

The present inventors examined methods for producing optically active alcohols with high optical purity, not by a resolution technique which gives a theoretical yield of less than 50%, but by reducing a ketone, where 100% of the raw material can be used, by an asymmetric reduction reaction using a highly stereoselective enzyme.

The present inventors used various types of enzymes that asymmetrically reduce a carbonyl with 1,1,1-trifluoroacetone (hereinafter abbreviated as TFAC) represented by formula (1) as the substrate, and examined methods for synthesizing an optically active 1,1,1-trifluoro-2-propanol (hereinafter abbreviated as TFIP) represented by formula (2) or (3). As a result, the present inventors succeeded in discovering that every one of the following produces (S)-TFIP with very high stereoselectivity of 90% e.e. or more:
alcohol dehydrogenase CpSADH (a protein comprising the amino acid sequence of SEQ ID NO: 9) derived from *Candida parapsilosis*;
alcohol dehydrogenase ReSADH (a protein comprising the amino acid sequence of SEQ ID NO: 10) derived from *Rhodococcus erythropolis;*
carbonyl reductase ScoPAR (a protein comprising the amino acid sequence of SEQ ID NO: 11) derived from *Streptomyces coelicolor;*
(2S,3S)-butanediol dehydrogenase ZraSBDH (a protein comprising the amino acid sequence of SEQ ID NO: 12) derived from *Zoogloea ramigera*;
carbonyl reductase ScGCY1 (a protein comprising the amino acid sequence of SEQ ID NO: 13) derived from *Saccharomyces cerevisiae;*
tropinone reductase HnTR1 (a protein comprising the amino acid sequence of SEQ ID NO: 14) derived from *Hyoscyamus niger;*
tropinone reductase DsTR1 (a protein comprising the amino acid sequence of SEQ ID NO: 15) derived from *Datura stramonium;* and
alcohol dehydrogenase BstADHT (a protein comprising the amino acid sequence of SEQ ID NO: 16) derived from *Geobacillus stearothermophilus.* Furthermore, the present inventors succeeded in discovering that alcohol dehydrogenase PfODH (a protein comprising the amino acid sequence of SEQ ID NO: 18) derived from *Pichia finlandica* produces (R)-TFIP with very high stereoselectivity of 95% e.e. or more.

It had been known that CpSADH (Japanese Patent No. 3574682) produces (S)-1,3-butanediol by asymmetric reduction of 4-hydroxy-2-butanone, ReSADH (Appl. Microbiol. Biotechnol., 62, 380-386 (2003)) and ScoPAR (Japanese Patent Application Kokai Publication No. (JP-A) 2005-95022 (unexamined, published Japanese patent application)) produces (S)-2-octanol by asymmetric reduction of 2-octanone, ZraSBDH (JP-A (Kokai) 2004-357639) produces (2S,3S)-butanediol by asymmetric reduction of 2,3-butanedione, ScGCY1 (FEBS Lett., 238, 123-128, (1988)) produces ethyl (S)-hydroxybutanoate by asymmetric reduction of ethyl acetoacetate, HnTR1 and DsTR1 (both described in JP-A (Kokai) 2003-230398) produce tropine by asymmetric reduction of tropinone, BstADHT (FEBS Lett., 33, 1-3, (1973)) produces ethanol by reducing acetaldehyde, and PfODH (WO 01-061014) produces (R)-2-octanol by asymmetric reduction of 2-octanone. However, whether these enzymes have activity towards TFAC, and if they do have activity, with what degree of stereoselectivity they will reduce TFAC, and whether they will produce the (S) or (R)-type configuration were impossible to predict. Therefore, discovering that (S)-TFIP or (R)-TFIP is produced with high selectivity of 90% e.e. or more was a surprising result.

When various enzymes that act on ketones, for example, alcohol dehydrogenase ScADH1 and ScADH2 derived from *Saccharomyces cerevisiae* (Arch. Biochem. Biophys., 126, 933-944 (1968)) and carbonyl reductase ScGRE3 derived from *Saccharomyces cerevisiae* (J. Org. Chem., 63, 4996-5000, (1998)) were made to exhibit their effects, the optical purities of the obtained (S)-TFIP were 82.3% e.e., 58.0% e.e., and 69.6% e.e., respectively.

The present invention was achieved in view of the above circumstances, and provides the following [1] to [6]:
[1] a method for producing (S)-1,1,1-trifluoro-2-propanol represented by formula (2), 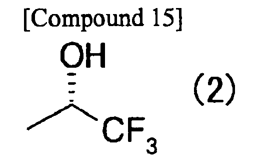 which comprises the step of reacting a protein encoded by the polynucleotide of any one of the following (a) to (e), a microorganism or a transformant strain that functionally expresses said protein, or a processed material thereof, with 1,1,1-trifluoroacetone represented by formula (1): 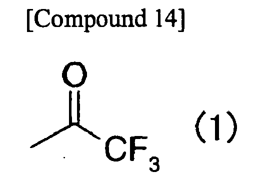
   (a) a polynucleotide comprising the nucleotide sequence of any one of SEQ ID NOs: 1 to 8;
   (b) a polynucleotide encoding a protein comprising the amino acid sequence of any one of SEQ ID NOs: 9 to 16;
   (c) a polynucleotide encoding a protein comprising amino acids with one or more amino acid substitutions, deletions, insertions, and/or additions in the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
   (d) a polynucleotide that hybridizes under stringent conditions with a DNA comprising the nucleotide sequence of any one of SEQ ID NOs: 1 to 8, wherein the polynucleotide encodes a protein having an activity of reducing 1,1,1-trifluoroacetone represented by formula (1) to produce (S)-1,1,1-trifluoro-2-propanol represented by formula (2); and
   (e) a polynucleotide encoding a protein comprising an amino acid sequence having 80% or more homology to the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
[2] a method for producing (R)-1,1,1-trifluoro-2-propanol represented by formula (3), 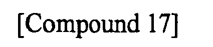 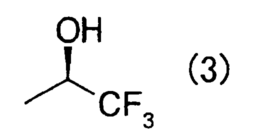 which comprises the step of reacting a protein encoded by the polynucleotide of any one of the following (a) to (e), a microorganism or a transformant strain that functionally expresses said protein, or a processed material thereof, with 1,1,1-trifluoroacetone represented by formula (1): 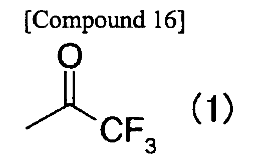
   (a) a polynucleotide comprising the nucleotide sequence of SEQ ID NO: 17;
   (b) a polynucleotide encoding a protein comprising the amino acid sequence of SEQ ID NO: 18;
   (c) a polynucleotide encoding a protein comprising amino acids with one or more amino acid substitutions, deletions, insertions, and/or additions in the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
   (d) a polynucleotide that hybridizes under stringent conditions with a DNA comprising the nucleotide sequence of SEQ ID NO: 17, wherein the polynucleotide encodes a protein having an activity of reducing 1,1,1-trifluoroacetone represented by formula (1) to produce (R)-1,1,1-trifluoro-2-propanol represented by formula (3); and
   (e) a polynucleotide encoding a protein comprising an amino acid sequence having 80% or more homology to the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
[3] a method for producing (S)-1,1,1-trifluoro-2-propanol represented by formula (2), 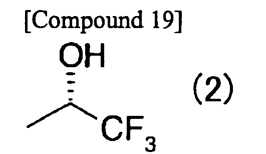 which comprises the step of reacting a protein encoded by the polynucleotide of any one of the following (a) to (e), a transformant strain that coexpresses a coenzyme corresponding to said protein and a dehydrogenase having an activity to regenerate reduced nicotinamide adenine dinucleotide (NADH) or reduced nicotinamide adenine dinucleotide phosphate (NADPH), or a processed material thereof, with 1,1,1-trifluoroacetone represented by formula (1): 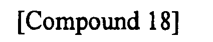 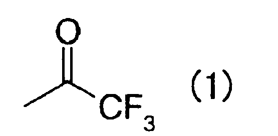
   (a) a polynucleotide comprising the nucleotide sequence of any one of SEQ ID NOs: 1 to 8;
   (b) a polynucleotide encoding a protein comprising the amino acid sequence of any one of SEQ ID NOs: 9 to 16;
   (c) a polynucleotide encoding a protein comprising amino acids with one or more amino acid substitutions, deletions, insertions, and/or additions in the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
   (d) a polynucleotide that hybridizes under stringent conditions with a DNA comprising the nucleotide sequence of any one of SEQ ID NOs: 1 to 8, wherein the polynucleotide encodes a protein having an activity of reducing 1,1,1-trifluoroacetone represented by formula (1) to produce (S)-1,1,1-trifluoro-2-propanol represented by formula (2); and
   (e) a polynucleotide encoding a protein comprising an amino acid sequence having 80% or more homology to the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
[4] a method for producing (R)-1,1,1-trifluoro-2-propanol represented by formula (3), 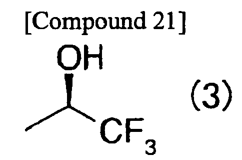 which comprises the step of reacting a protein encoded by the polynucleotide of any one of the following (a) to (e), a transformant strain that coexpresses a coenzyme corresponding to said protein and a dehydrogenase having an activity to regenerate reduced nicotinamide adenine dinucleotide (NADH) or reduced nicotinamide adenine dinucleotide phosphate (NADPH,), or a processed material thereof, with 1,1,1-trifluoroacetone represented by formula (1): 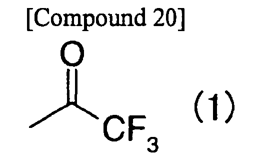
   (a) a polynucleotide comprising the nucleotide sequence of SEQ ID NO: 17;
   (b) a polynucleotide encoding a protein comprising the amino acid sequence of SEQ ID NO: 18;
   (c) a polynucleotide encoding a protein comprising amino acids with one or more amino acid substitutions, deletions, insertions, and/or additions in the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
   (d) a polynucleotide that hybridizes under stringent conditions with a DNA comprising the nucleotide sequence of SEQ ID NO: 17, wherein the polynucleotide encodes a protein having an activity of reducing 1,1,1-trifluoroacetone represented by formula (1) to produce (R)-1,1,1-trifluoro-2-propanol represented by formula (3); and
   (e) a polynucleotide encoding a protein comprising an amino acid sequence having 80% or more homology to the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
[5] the method of [3] or [4], wherein the dehydrogenase having an activity to regenerate reduced nicotinamide adenine dinucleotide (NADH) or reduced nicotinamide adenine dinucleotide phosphate (NADPH) is glucose dehydrogenase or formate dehydrogenase; and
[6] a method for stably producing (S)-1,1,1-trifluoro-2-propanol represented by formula (2) 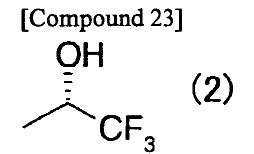 with an optical purity of 99.5% e.e. or more by reacting a protein encoded by the polynucleotide of any one of the following (a) to (e), a microorganism or a transformant strain that functionally expresses said protein, or a processed material thereof, with 1,1,1-trifluoroacetone represented by formula (1) within a pH range of 5.0 to 6.4: 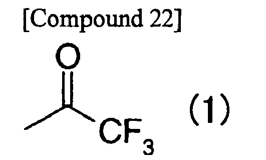
   (a) a polynucleotide comprising the nucleotide sequence of SEQ ID NO: 1;
   (b) a polynucleotide encoding a protein comprising the amino acid sequence of SEQ ID NO: 9;
   (c) a polynucleotide encoding a protein comprising amino acids with one or more amino acid substitutions, deletions, insertions, and/or additions in the amino acid sequence of SEQ ID NO: 9, wherein the protein has an activity of reducing 1,1,1-trifluoroacetone represented by formula (1) to produce (S)-1,1,1-trifluoro-2-propanol represented by formula (2);
   (d) a polynucleotide that hybridizes under stringent conditions with a DNA comprising the nucleotide sequence of SEQ ID NO: 1, wherein the polynucleotide encodes a protein having an activity of reducing 1,1,1-trifluoroacetone represented by formula (1) to produce (S)-1,1,1-trifluoro-2-propanol represented by formula (2); and
   (e) a polynucleotide encoding a protein comprising an amino acid sequence having 80% or more homology to the amino acid sequence of SEQ ID NO: 9, wherein the protein has an activity of reducing 1,1,1-trifluoroacetone represented by formula (1) to produce (S)-1,1,1-trifluoro-2-propanol represented by formula (2).

### Brief Description of the Drawings

Fig. 1 depicts the restriction enzyme map of plasmid pSF-CPA4 constructed in Example 1. In the figure, CpSADH refers to the *Candida parapsilosis*-derived alcohol dehydrogenase gene, McFDH refers to the *Mycobacterium* vaccae-derived formate dehydrogenase gene, lacI^{q} refers to lac repressor, P(trc) refers to trc promoter, mc refers to multicloning site, T(rrnB) refers to rrnB terminator, amp refers to ampicillin resistance gene, ori refers to replication origin, and rop refers to rop protein gene.
Fig. 2 depicts the restriction enzyme map of plasmid pSE-RED1 constructed in Example 4. ReSADH refers to the *Rhodococcus erythropolis*-derived alcohol dehydrogenase gene.
Fig. 3 depicts the restriction enzyme map of plasmid pSF-RED1 constructed in Example 5.
Fig. 4 depicts the restriction enzyme map of plasmid pSE-SCP7 constructed in Example 6. ScoPAR refers to the *Streptomyces coelicolor*-derived carbonyl reductase gene.
Fig. 5 depicts the restriction enzyme map of plasmid pSF-SCP7 constructed in Example 8.
Fig. 6 depicts the restriction enzyme map of plasmid pSE-GCY1 constructed in Example 11. ScGCY refers to the *Saccharomyces cerevisiae*-deriνed carbonyl reductase gene.
Fig. 7 depicts the restriction enzyme map of plasmid pSG-GCY1 constructed in Example 12. BsGDH refers to the *Bacillus subtilis-*derived glucose dehydrogenase gene.
Fig. 8 depicts the restriction enzyme map of plasmid pSE-BSA1 constructed in Example 14. BstADHT refers to the *Geobacillus thermocatenulas*-derived alcohol dehydrogenase gene.
Fig. 9 depicts the restriction enzyme map of plasmid pSU-SCA1 constructed in Example 16. ScADH1 refers to the *Saccharomyces cerevisiae*-derived alcohol dehydrogenase I gene.
Fig. 10 depicts the restriction enzyme map of plasmid pSU-SCA2 constructed in Example 18. ScADH2 refers to the *Saccharomyces cerevisiae*-derived alcohol dehydrogenase II gene.
Fig. 11 depicts the restriction enzyme map of plasmid pSE-GRE3 constructed in Example 20. ScGRE3 refers to the *Saccharomyces cerevisiae*-derived carbonyl reductase gene.

### Mode for Carrying Out the Invention

The present invention relates to methods for enzymatically producing optically active (S)-1,1,1-trifluoro-2-propanol. The present invention is based on the discovery made by the present inventors that (S)-1,1,1-trifluoro-2-propanol represented by formula (2) 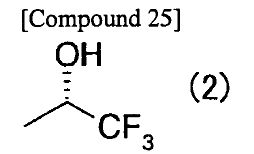 can be produced efficiently by reacting an enzyme of the present invention, a microorganism or a transformant strain that functionally expresses the enzyme, or a processed substance thereof, with 1,1,1-trifluoroacetone represented by formula (1).

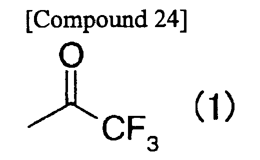

Enzymes that catalyze the above-mentioned reaction from formula (1) to formula (2) include:
alcohol dehydrogenase CpSADH,
alcohol dehydrogenase ReSADH,
carbonyl reductase ScoPAR,
(2S,3S)-butanediol dehydrogenase ZraSBDH,
carbonyl reductase ScGCY1,
tropinone reductase HnTR1,
tropinone reductase DsTR1, and
alcohol dehydrogenase BstADHT.

The present invention further relates to methods for enzymatically producing optically active (R)-1,1,1-trifluoroacetone. This invention is based on the discovery made by the present inventors that (R)-1,1,1-trifluoro-2-propanol represented by formula (3) 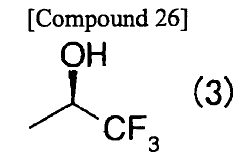 can be produced efficiently by reacting an enzyme of the present invention, a microorganism or a transformant strain that functionally expresses the enzyme, or a processed substance thereof, with 1,1,1-trifluoroacetone represented by formula (1).

Herein, enzymes that catalyze the above-mentioned reaction from formula (1) to formula (3) include alcohol dehydrogenase PfODH. In the present specification, enzymes that catalyze the reaction from formula (1) to formula (2), and enzymes that catalyze the reaction from formula (1) to formula (3) are referred to as "enzymes having carbonyl reductase activity".

### Enzymes

Hereafter, enzymes of the present invention (alcohol dehydrogenase CpSADH, alcohol dehydrogenase ReSADH, carbonyl reductase ScoPAR, (2S,3S)-butanediol dehydrogenase ZraSBDH, carbonyl reductase ScGCY1, tropinone reductase HnTR1, tropinone reductase DsTR1, alcohol dehydrogenase BstADHT, and alcohol dehydrogenase PfODH) will be described.

First, alcohol dehydrogenase CpSADH in the present invention includes proteins comprising the amino acid sequence of SEQ ID NO: 9. A nucleotide sequences encoding this amino acid sequence include the nucleotide sequence of SEQ ID NO: 1. Alcohol dehydrogenase CpSADH of the present invention includes proteins encoded by:
(1) a polynucleotide encoding a protein comprising amino acids with one or more amino acid substitutions, deletions, insertions, and/or additions in the amino acid sequence of SEQ ID NO: 9, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 1, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 9, wherein the protein has an activity of reducing 1,1,1-trifluoroacetone represented by formula (1) to produce (S)-1,1,1-trifluoro-2-propanol represented by formula (2).

Carbonyl reductase CpSADH used in the present invention can be prepared from *Candida parapsilosis* by the method described in Japanese Patent No. 3574682. Alternatively, the enzyme can be obtained from a recombinant by cloning, from *Candida parapsilosis* or such by PCR, the DNA of SEQ ID NO: 1 which encodes the *Candida parapsilosis*-derived CpSADH, and then expressing CpSADH using a recombinant prepared by introducing the DNA in an expressible form to a heterologous host, such as *E. coli.*

The enzyme activity of CpSADH in the present invention can be measured, for example, as described below but it is not limited thereto. More specifically, an example includes a method in which a reaction is carried out at 30°C in a reaction solution containing 100 mM Tris-HCl buffer (pH 9.0), 2.5 mM NAD⁺, 50 mM (S)-1,3-butanediol, and the enzyme and the increase in absorbance at 340 nm, resulting from the production of NADH, is measured. In this case, 1 U is defined as the amount of enzyme that catalyzes the production of 1 µmol of NADH in one minute.

Next, alcohol dehydrogenase ReSADH will be described. Alcohol dehydrogenase ReSADH in the present invention includes proteins comprising the amino acid sequence of SEQ ID NO: 10, and nucleotide sequences encoding this amino acid sequence include the nucleotide sequence of SEQ ID NO: 2. Alcohol dehydrogenase ReSADH of the present invention includes proteins encoded by:
(1) a polynucleotide encoding a protein comprising amino acids with one or more amino acid substitutions, deletions, insertions, and/or additions in the amino acid sequence of SEQ ID NO: 10, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 2, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 10, wherein the protein has an activity of reducing 1,1,1-trifluoroacetone represented by formula (1) to produce (S)-1,1,1-trifluoro-2-propanol represented by formula (2).

Alcohol dehydrogenase ReSADH used in the present invention can be prepared from *Rhodococcus erythropolis* by the method described in Appl. Microbiol. Biotechnol., 62, 380-386 (2003). Alternatively, the enzyme can be obtained from a recombinant by cloning from *Rhodococcus erythropolis* or such by PCR the DNA of SEQ ID NO: 2 which encodes the *Rhodococcus erythropolis*-derived ReSADH, and then expressing ReSADH using a recombinant prepared by introducing the DNA in an expressible form to a heterologous host, such as *E. coli.*

The enzyme activity of ReSADH in the present invention can be measured, for example, as described below but it is not limited thereto. More specifically, a reaction is carried out at 30°C in a reaction solution containing 100 mM Tris-HCl buffer (pH 9.0), 2.5 mM NAD⁺, 5 mM (S)-2-octanol, and the enzyme and the increase in absorbance at 340 nm, resulting from the production of NADH, is measured. In this case, 1 U is defined as the amount of enzyme that catalyzes the production of 1 µmol of NADH in one minute.

Next, carbonyl reductase ScoPAR will be described. Carbonyl reductase ScoPAR in the present invention includes proteins comprising the amino acid sequence of SEQ ID NO: 11. Nucleotide sequences encoding this amino acid sequence include the nucleotide sequence of SEQ ID NO: 3. Carbonyl reductase ScoPAR of the present invention includes proteins encoded by:
(1) a polynucleotide encoding a protein comprising amino acids with one or more amino acid substitutions, deletions, insertions, and/or additions in the amino acid sequence of SEQ ID NO: 11, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 3, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 11, wherein the protein has an activity of reducing 1,1,1-trifluoroacetone represented by formula (1) to produce (S)-1,1,1-trifluoro-2-propanol represented by formula (2).

Carbonyl reductase ScoPAR used in the present invention can be prepared from *Streptomyces coelicolor* by the method described in JP-A (Kokai) 2005-95022. Alternatively, the enzyme can be obtained from a recombinant by cloning from *Streptomyces coelicolor* or such by PCR the DNA of SEQ ID NO: 3 which encodes the *Streptomyces coelicolor*-derived ScoPAR, and then expressing ScoPAR using a recombinant prepared by introducing the DNA in an expressible form to a heterologous host, such as *E. coli.*

The enzyme activity of ScoPAR in the present invention can be measured, for example, as described below but it is not limited thereto. More specifically, a reaction is carried out at 30°C in a reaction solution containing 100 mM Tris-HCI buffer (pH 9.0), 2.5 mM NAD⁺, 5 mM (S)-2-octanol, and the enzyme and the increase in absorbance at 340 nm, resulting from the production of NADH, is measured. 1 U is defined as the amount of enzyme that catalyzes the production of 1 µmol of NADH in one minute.

Next, (2S,3S)-butanediol dehydrogenase ZraSBDH will be described. (2S,3S)-butanediol dehydrogenase ZraSBDH in the present invention includes proteins comprising the amino acid sequence of SEQ ID NO: 12, and nucleotide sequences encoding this amino acid sequence include the nucleotide sequence of SEQ ID NO: 4. (2S,3S)-butanediol dehydrogenase ZraSBDH of the present invention includes proteins encoded by the following (1) to (3):
(1) a polynucleotide encoding a protein comprising amino acids with one or more amino acid substitutions, deletions, insertions, and/or additions in the amino acid sequence of SEQ ID NO: 12, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 4, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 12, wherein the protein has an activity of reducing 1,1,1-trifluoroacetone represented by formula (1) to produce (S)-1,1,1-trifluoro-2-propanol represented by formula (2).

(2S,3S)-butanediol dehydrogenase ZraSBDH used in the present invention can be prepared from *Zoogloea ramigera* by the method described in JP-A (Kokai) 2004-357639. Alternatively, the enzyme can be obtained from a recombinant by cloning from *Zoogloea ramigera* or such by PCR the DNA of SEQ ID NO: 4 which encodes the *Zoogloea ramigera*-derived ZraSBDH, and then expressing ZraSBDH using a recombinant prepared by introducing the DNA in an expressible form to a heterologous host, such as *E. coli.*

The enzyme activity of ZraSBDH in the present invention can be measured, for example, as described below but it is not limited thereto. More specifically, a reaction is carried out at 30°C in a reaction solution containing 100 mM phosphate buffer (pH 8.0), 2.5 mM NAD⁺, 50 mM (2S,3S)-butanediol, and the enzyme, and the increase in absorbance at 340 nm, resulting from the production of NADH, is measured. 1 U is defined as the amount of enzyme that catalyzes the production of 1 µmol of NADH in one minute.

Next, carbonyl reductase ScGCY1 will be described. Carbonyl reductase ScGCY1 in the present invention includes proteins comprising the amino acid sequence of SEQ ID NO: 13. Nucleotide sequences encoding this amino acid sequence include the nucleotide sequence of SEQ ID NO: 5. Carbonyl reductase ScGCY1 of the present invention includes proteins encoded by:
(1) a polynucleotide encoding a protein comprising amino acids with one or more amino acid substitutions, deletions, insertions, and/or additions in the amino acid sequence of SEQ ID NO: 13, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 5, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 13, wherein the protein has an activity of reducing 1,1,1-trifluoroacetone represented by formula (1) to produce (S)-1,1,1-trifluoro-2-propanol represented by formula (2).

Carbonyl reductase ScGCY1 used in the present invention can be obtained from a recombinant by cloning from *Saccharomyces cerevisiae* or such by PCR the DNA of SEQ ID NO: 5 which encodes the *Saccharomyces cerevisiae*-derived ScGCY1, and then expressing ScGCY1 using a recombinant prepared by introducing the DNA in an expressible form to a heterologous host, such as E. *coli.*

The enzyme activity of ScGCY1 in the present invention can be measured, for example, as described below but it is not limited thereto. More specifically, a reaction is carried out at 30°C in a reaction solution containing 100 mM phosphate buffer (pH 6.5), 0.2 mM NADPH, 20 mM ethyl acetoacetate, and the enzyme, and the decrease in absorbance at 340 nm, resulting from the decrease of NADPH, is measured. 1 U is defined as the amount of enzyme that catalyzes the decrease of 1 µmol of NADH in one minute.

Next, tropinone reductase HnTR1 will be described. Tropinone reductase HnTR1 in the present invention includes proteins comprising the amino acid sequence of SEQ ID NO: 14. Nucleotide sequences encoding this amino acid sequence include the nucleotide sequence of SEQ ID NO: 6. Tropinone reductase HnTR1 of the present invention includes proteins encoded by:
(1) a polynucleotide encoding a protein comprising amino acids with one or more amino acid substitutions, deletions, insertions, and/or additions in the amino acid sequence of SEQ ID NO: 14, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 6, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 14, wherein the protein has an activity of reducing 1,1,1-trifluoroacetone represented by formula (1) to produce (S)-1,1,1-trifluoro-2-propanol represented by formula (2).

Tropinone reductase HnTR1 used in the present invention can be prepared from *Hyoscyamus niger* by the method described in JP-A (Kokai) 2003-230398. Alternatively, the enzyme can be obtained from a recombinant by cloning from *Hyoscyamus niger* or such by PCR the DNA of SEQ ID NO: 6 which encodes the *Hyoscyamus niger*-derived HnTR1, and then expressing HnTR1 using a recombinant prepared by introducing the DNA in an expressible form to a heterologous host, such as *E. coli.*

The enzyme activity of HnTR1 in the present invention can be measured, for example, as described below but it is not limited thereto. More specifically, a reaction is carried out at 30°C in a reaction solution containing 100 mM phosphate buffer (pH 6.5), 0.2 mM NADPH, 4 mM tropinone, and the enzyme, and the decrease in absorbance at 340 nm, resulting from the decrease of NADPH is measured. 1 U is defined as the amount of enzyme that catalyzes the decrease of 1 µmol of NADH in one minute.

Next, tropinone reductase DsTR1 will be described. Tropinone reductase DsTR1 in the present invention includes proteins comprising the amino acid sequence of SEQ ID NO: 15. Nucleotide sequences encoding this amino acid sequence include the nucleotide sequence of SEQ ID NO: 7. Tropinone reductase DsTR1 of the present invention includes proteins encoded by:
(1) a polynucleotide encoding a protein comprising amino acids with one or more amino acid substitutions, deletions, insertions, and/or additions in the amino acid sequence of SEQ ID NO: 15, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 7, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 15, wherein the protein has an activity of reducing 1,1,1-trifluoroacetone represented by formula (1) to produce (S)-1,1,1-trifluoro-2-propanol represented by formula (2).

Tropinone reductase DsTR1 used in the present invention can be prepared from *Datura stramonium* by the method described in JP-A (Kokai) 2003-230398. Alternatively, the enzyme can be obtained from a recombinant by cloning from *Datura stramonium* or such by PCR the DNA of SEQ ID NO: 7 which encodes the *Datura stramonium*-derived DsTR1, and then expressing DsTR1 using a recombinant prepared by introducing the DNA in an expressible form to a heterologous host, such as *E. coli.*

The enzyme activity of DsTR1 in the present invention can be measured, for example, as described below but it is not limited thereto. More specifically, a reaction is carried out at 30°C in a reaction solution containing 100 mM phosphate buffer (pH 6.5), 0.2 mM NADPH, 4 mM tropinone, and the enzyme, and the decrease in absorbance at 340 nm resulting from the decrease of NADPH is measured. 1 U is defined as the amount of enzyme that catalyzes the decrease of 1 µmol of NADH in one minute.

Next, alcohol dehydrogenase BstADHT will be described. Alcohol dehydrogenase BstADHT in the present invention includes proteins comprising the amino acid sequence of SEQ ID NO: 16. Nucleotide sequences encoding this amino acid sequence include the nucleotide sequence of SEQ ID NO: 8. Alcohol dehydrogenase BstADHT of the present invention includes proteins encoded by:
(1) a polynucleotide encoding a protein comprising amino acids with one or more amino acid substitutions, deletions, insertions, and/or additions in the amino acid sequence of SEQ ID NO: 16, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 8, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 16, wherein the protein has an activity of reducing 1,1,1-trifluoroacetone represented by formula (1) to produce (S)-1,1,1-trifluoro-2-propanol represented by formula (2).

Alcohol dehydrogenase BstADHT used in the present invention can be obtained from a recombinant by cloning from *Geobacillus stearothermophilus* or such by PCR the DNA of SEQ ID NO: 8 which encodes the *Geobacillus stearothermophilus*-derived BstADHT, and then expressing BstADHT using a recombinant prepared by introducing the DNA in an expressible form to a heterologous host, such as *E. coli.*

The enzyme activity of BstADHT in the present invention can be measured, for example, as described below but it is not limited thereto. More specifically, a reaction is carried out at 30°C in a reaction solution containing 100 mM phosphate buffer (pH 8.0), 2.5 mM NAD⁺, 100 mM ethanol, and the enzyme, and the increase in absorbance at 340 nm resulting from the production of NADH, is measured. 1 U is defined as the amount of enzyme that catalyzes the production of 1 µmol of NADH in one minute.

Finally, alcohol dehydrogenase PfODH will be described. Alcohol dehydrogenase PfODH in the present invention includes proteins comprising the amino acid sequence of SEQ ID NO: 18. Nucleotide sequences encoding this amino acid sequence include the nucleotide sequence of SEQ ID NO: 17. Alcohol dehydrogenase PfODH of the present invention includes proteins encoded by:
(1) a polynucleotide encoding a protein comprising amino acids with one or more amino acid substitutions, deletions, insertions, and/or additions in the amino acid sequence of SEQ ID NO: 18, wherein the protein has an activity of reducing 1,1,1-trifluoroacetone represented by formula (1) to produce (S)-1,1,1-trifluoro-2-propanol represented by formula (2);
(2) a polynucleotide that hybridizes under stringent conditions with a DNA comprising the nucleotide sequence of SEQ ID NO: 17, wherein the polynucleotide encodes a protein having an activity of reducing 1,1,1-trifluoroacetone represented by formula (1) to produce (S)-1,1,1-trifluoro-2-propanol represented by formula (2); and
(3) a polynucleotide encoding a protein comprising an amino acid sequence having 80% or more homology to the amino acid sequence of SEQ ID NO: 18, wherein the protein has an activity of reducing 1,1,1-trifluoroacetone represented by formula (1) to produce (S)-1,1,1-trifluoro-2-propanol represented by formula (2).

Alcohol dehydrogenase PfODH used in the present invention can be prepared from *Pichia finlandica* by the method described in WO 01/061014. Alternatively, the enzyme can be obtained from a recombinant by cloning from *Pichia finlandica* or such by PCR the DNA of SEQ ID NO: 17 which encodes the *Pichia finlandica*-derived PfODH, and then expressing PfODH using a recombinant prepared by introducing the DNA in an expressible form to a heterologous host, such as *E. coli.*

The enzyme activity of PfODH in the present invention can be measured, for example, as described below but it is not limited thereto. More specifically, a reaction is carried out at 30°C in a reaction solution containing 100 mM Tris-HCl buffer (pH 9.0), 2.5 mM NAD⁺, 5 mM (R)-2-octanol, and the enzyme, and the increase in absorbance at 340 nm, resulting from the production of NADH is measured. 1 U is defined as the amount of enzyme that catalyzes the production of 1 µmol of NADH in one minute.

The amino acid sequence with one or more (for example, 2 to 100, preferably 2 to 50, and more preferably 2, 3, 4, 5, 6, 7, 8, 9, or 10) amino acid deletions, substitutions, insertions, and/or additions in an above-mentioned enzyme of the present invention (a protein comprising the amino acid sequence of any one of SEQ ID NOs: 9 to 16 and 18) can be obtained by appropriately introducing a substitution, deletion, insertion, and/or addition of mutations to the polynucleotide of any one of SEQ ID NOs: 9 to 16 and 18 using site-directed mutagenesis (Nucleic Acid Res. 10, pp. 6487 (1982); Methods in Enzymol. 100, pp. 448 (1983), Molecular Cloning 2nd Ed., Cold Spring Harbor Laboratory Press (1989); PCR A Practical Approach IRL Press pp. 200 (1991)).

A polynucleotide of the present invention that can hybridize under stringent conditions with a polynucleotide comprising the nucleotide sequence of any one of SEQ ID NOs: 1 to 8 and 17 refers to a polynucleotide that hybridizes under the conditions described in the manual (for example, a wash at 42°C, primary wash buffer containing 0.5x SSC) when using, for example, ECL direct nucleic acid labeling and detection system (manufactured by Amersham Pharmacia Biotech) with a DNA prepared by selecting one or more sequences including at least 20, preferably at least 30, for example, 40, 60, or 100 arbitrary continuous nucleotides of the polynucleotide of any one of SEQ ID NOs: 1 to 8 and 17 as the probe DNA. More specifically, the term "stringent conditions" ordinarily refers to, for example, conditions of 42°C, 2x SSC, and 0.1% SDS, preferably conditions of 50°C, 2x SSC, and 0.1% SDS, and more preferably 65°C, 0.1x SSC, and 0.1% SDS, but is not particularly limited to these conditions. Multiple factors such as temperature and salt concentration can be considered as factors that affect the stringency of hybridization, and those skilled in the art can realize the most suitable stringency by appropriately selecting these factors.

Homologs of the polynucleotide in the present invention include a polynucleotide encoding a protein having homology of at least 80%, preferably at least 85%, more preferably 90%, 91%, 92%, 93%, or 94%, and even more preferably 95% or more (for example, 96%, 97%, 98%, or 99%) to the amino acid sequence of any one of SEQ ID NOs: 9 to 16 and 18. Protein homology searches can be performed, for example, against databases of amino acid sequences of proteins, such as SWISS-PROT, PIR, or DAD, databases of DNA sequences, such as DDBJ, EMBL, or Gene-Bank, or databases of amino acid sequences predicted from DNA sequences, by using programs such as BLAST and FASTA, for example, via the internet.

The form of the above-mentioned polynucleotides encoding enzymes of the present invention is not particularly limited so long as the polynucleotides can encode an enzyme of the present invention, and includes in addition to cDNA, genomic DNA and chemically synthesized DNA.

### Microorganisms or transformants that functionally express enzymes, or a processed material thereof

In the production method of the present invention, microorganisms that functionally express the above-mentioned enzymes or processed materials thereof may be used instead of the above-mentioned enzymes. Microorganisms of the present invention include isolated microorganisms expressing at least one of the above-mentioned enzymes. Without limitation, such microorganisms include, for example, *Candida parapsilosis, Rhodococcus erythropolis, Streptomyces coelicolor, Zoogloea ramigera, Saccharomyces cerevisiae, Hyoscyamus niger, Datura stramonium, Geobacillus stearothermophilus,* and *Pichia finlandica.*

Microorganisms of the present invention also include microorganisms that have been transformed with a vector comprising a DNA encoding at least one of the above-mentioned enzymes. Such microorganisms can be produced by the same method as that for producing the transformants described below.

To "functionally express" means to express an enzyme in a state in which its function is maintained. In the present invention, so long as the enzyme maintains its function, there are no limitations on the method for expressing the enzyme. Furthermore, in the present invention, the level of expression of the above-mentioned enzyme by the microorganism is also not limited. Therefore, as long as the above-mentioned enzyme is expressed, even if the amount is small, it is included in the microorganism of the present invention.

In the production methods of the present invention, instead of the above-mentioned enzyme, a transformant transformed by a vector comprising a DNA encoding the above-mentioned enzyme or a processed material thereof may be used.

A suitable vector when using a transformant transformed by a vector comprising a DNA encoding the above-mentioned enzyme, instead of the above-mentioned enzyme, includes various vectors such as plasmids, cosmids, viruses, and bacteriophages (see Molecular Cloning, A Laboratory Manual 2nd ed., Cold Spring Harbor Press (1989); Current Protocols in Molecular Biology, John Wiley & Sons (1987)). A preferred vector used in the present invention includes, for example, pK4EC prepared by inserting a gene encoding the above-mentioned enzyme of the present invention in an expressible manner to an E. *coli* expression vector pSE420D, but is not limited thereto.

The aforementioned vector preferably includes all components of the regulatory sequence necessary for expression of the inserted DNA. Furthermore, the vector may include a selection marker for selection of host cells introduced with the vector.

In the present invention, a sequence encoding a signal peptide can be added to the DNA. Addition of a signal peptide enables transfer of the protein expressed in the host cell to the lumen of the endoplasmic reticulum. Alternatively, when Gram-negative bacteria are used as a host, the protein expressed in the host cell can be transferred into the periplasm or transferred outside the cell using a signal peptide. One can use any signal peptide that can function in the host cell that will be used. Therefore, a signal peptide derived from a cell heterologous to the host cell can also be used. Furthermore, as necessary, a linker, a start codon (ATG), a stop codon (TAA, TAG, or TGA), and such can be added when introducing the DNA into the vector.

The microorganisms transformed to express respective enzymes in the present invention are not particularly limited so long as they are organisms that can be transformed by a recombinant vector comprising a polynucleotide encoding the respective enzyme, and can exhibit its activity. Examples of microorganisms that can be used include the following microorganisms:
Bacteria for which host-vector systems have been developed, such as:
   the genus *Escherichia*,
   the genus *Bacillus,*
   the genus *Pseudomonas,*
   the genus *Serratia,*
   the genus *Brevibacterium,*
   the genus *Corynebacterium,*
   the genus *Streptococcus,* and
   the genus *Lactobacillus;*

Actinomycetes for which host-vector systems have been developed, such as:
the genus *Rhodococcus,* and
the genus *Streptomyces;*

Yeasts for which host-vector systems have been developed, such as:
the genus *Saccharomyces,*
the genus *Kluyveromyces,*
the genus *Schizosaccharomyces,*
the genus *Zygosaccharomyces*,
the genus *Yarrowia*,
the genus *Trichosporon,*
the genus *Rhodosporidium*,
the genus *Pichia,* and
the genus *Candida;* and

Molds for which host-vector systems have been developed, such as:
the genus *Neurospora,*
the genus *Aspergillus,*
the genus *Cephalosporium,* and
the genus *Trichoderma.*

Procedures for production of transformants and construction of recombinant vectors compatible to the hosts can be carried out by following techniques commonly used in the field of molecular biology, bioengineering, and genetic engineering (for example, Sambrook *et al.,* Molecular Cloning, Cold Spring Harbor Laboratories). For expression of the carbonyl reductase gene of the present invention whose electron donor is NADPH in a microorganism or such, first, the DNA is introduced into a plasmid vector or a phage vector that will stably exist in the microorganism, and then this genetic information needs to be transcribed and translated. To accomplish this, a promoter which corresponds to the regulatory unit for transcription and translation can be incorporated at the 5'-side (upstream) of the DNA strand of the present invention, and more preferably a terminator is incorporated at the 3'-side (downstream). Such promoters and terminators need to be those known to function in the microorganism to be used as the host. Such vectors, promoters, terminators, and such that can be used with various types of microorganisms are described in detail in "Biseibutsu-gaku Kiso Koza 8 (Basic Microbiological Seminar 8) Idenshi Kogaku (Genetic Engineering),, Kyoritsu Shuppan", and in particular, those that can be used with yeast are described in Adv. Biochem. Eng., 43, 75-102 (1990), and Yeast, 8, 423-488 (1992).

A variety of host and vector systems have been developed using plants and animals in addition to microorganisms. In particular, systems for expressing a large amount of heterologous proteins in insects using silkworms (Nature, 315, 592-594 (1985)) and in plants such as rapeseed, corn, or potato have been developed, and can be used suitably.

Introduction of DNA into a vector can be performed by ligase reactions using restriction enzyme sites (Current Protocols in Molecular Biology, John Wiley & Sons (1987) Section 11.4-11.11; Molecular Cloning, A Laboratory Manual 2nd ed., Cold Spring Harbor Press (1989) Section 5.61-5.63). By taking into consideration the frequency of codon usage of the host to be used, vectors with high expression efficiency can be designed by modifying the polynucleotide sequence as necessary (Grantham et al., Nucleic Acids Res. (1981) 9:r43-74).

As described above, various cells have been established as host cell strains. Methods for introducing expression vectors suitable for each cell line are also known, and those skilled in the art can select an introduction method suitable for each of the selected host cells. For example, transformation by calcium treatment, electroporation, and such are known for prokaryotic cells. Methods using agrobacterium are known for plant cells, and calcium phosphate precipitation method is an example for mammalian cells. The present invention is not particularly limited to these methods, and depending on the selected host, expression vectors can be introduced by various other known methods such as those using nuclear microinjection, protoplast fusion, DEAE-dextran method, cell fusion, electroporation, lipofectamine method (GIBCO BRL), and FuGENE6 reagent (Boehringer-Mannheim).

By culturing transformants transformed with a recombinant vector carrying the DNA as described above, proteins having the activity of reducing 1,1,1-trifluoroacetone to produce an optically active 1,1,1-trifluoro-2-propanol can be produced.

Methods for culturing the transformants are not particularly limited, and desirably, conditions such as medium, temperature, and time, which are suitable for growth of each of the selected host cell and most suitable for production of an enzyme of the present invention are selected. Enzymes can be purified from transformants by methods known to those skilled in the art. For example, transformants are grown in a medium suitable for growth of the host, and after sufficient growth, the bacterial cells are collected, the cells are homogenized in a buffer added with a reducing agent such as 2-mercaptoethanol or phenyl methane sulfonyl fluoride, or a protease inhibitor, and a cell-free extract is prepared. Enzymes can be purified from the cell-free extract by suitably combining fractionation based on solubility of the protein (precipitation by organic solvents, salting-out by ammonium sulfate, or the like), cation exchange chromatography, anion exchange chromatography, gel filtration, hydrophobic chromatography, affinity chromatography using chelates, pigments, or antibodies, or such.

In a preferred embodiment for producing optically active alcohols represented by formulas (2) and (3) described in the present invention, optically active alcohols can be produced by contacting the reaction solution with the enzyme molecule, processed material thereof, cultured material containing the enzyme molecule, or microorganisms or transformants that produce (express) the enzyme, or a processed material thereof to carry out the desired enzyme reaction. The manner in which the enzyme and the reaction solution are contacted is not limited to these specific examples.

The processed material used in the present invention refers to a product obtained by performing physical treatment, biochemical treatment, chemical treatment, or such to a biological cell. Biological cells include the above-mentioned plant cells carrying the enzyme as well as transformants that retain a gene of this enzyme in an expressible manner. Physical treatment for obtaining the processed material includes treatments such as freeze-thawing, sonication, pressurization, osmotic pressure difference, or grinding. Specific examples of biochemical treatments include treatment with a cell-wall digesting enzyme such as lysozyme. Furthermore, a chemical treatment includes treatment by contact with a surfactant, or an organic solvent such as toluene, xylene, or acetone. Processed materials include microorganisms whose cell membrane permeability has been changed by such treatment, cell-free extracts prepared by homogenizing bacterial cells by treatment with glass beads or enzymes, partially purified material thereof, or such.

Biological cells or processed materials used in the present invention can be used after immobilization by known methods such as the polyacrylamide method, sulfur-containing polysaccharide gel method (such as κ-carrageenan gel method), alginic acid gel method, agar gel method, or ion exchange resin method.

### Coenzymes

A preferred embodiment of the present invention provides methods for producing (S)-1,1,1-trifluoro-2-propanol (hereinafter abbreviated as (S)-TFIP) by reacting a protein having carbonyl reductase activity, which is selected from CpSADH, ReSADH, ScoPAR, ZraSBDH, ScGCY1, HnTR1, DsTR1, or BstADHT, a microorganism or a transformant that produces (expresses) the enzyme or protein, or a processed material thereof with 1,1,1-trifluoroacetone (hereinafter abbreviated as TFAC). Furthermore, the present invention provides methods for producing (R)-1,1,1-trifluoro-2-propanol (hereinafter abbreviated as (R)-TFIP) by reacting a protein having carbonyl reductase activity (PfODH), a microorganism or a transformant that produces (expresses) the enzyme or protein, or a processed material thereof with TFAC.

In the production methods of the present invention, in addition to the protein (enzyme) having carbonyl reductase activity, a coenzyme corresponding to this enzyme, and a dehydrogenase having the activity to regenerate reduced nicotinamide adenine dinucleotide (NADH) or reduced nicotinamide adenine dinucleotide phosphate (NADPH) may be used simultaneously.

Regeneration of NAD(P)H from NAD(P)⁺ produced from NAD(P)H accompanying the above-mentioned reduction reaction, can be achieved by using the ability of a microorganism to reduce NAD(P)⁺ (glycolytic pathway, the pathway of methylotrophs to assimilate C1 compounds, etc.). The ability to reduce NAD(P)⁺ can be enhanced by adding glucose, ethanol, or such to the reaction system. The reduction reaction can be achieved by adding either a microorganism having the ability to produce NAD(P)H from NAD(P)⁺, or a processed material thereof or enzyme thereof, to the reaction system. For example, NAD(P)H can be regenerated using a microorganism containing glucose dehydrogenase, alcohol dehydrogenase, formate dehydrogenase, amino acid dehydrogenase, organic acid dehydrogenase (such as malate dehydrogenase), phosphite dehydrogenase, hydrogenase, or such, a processed material thereof, or a purified or partially purified enzyme. Such components constituting the required reactions for NAD(P)H regeneration may either be added to the reaction system to produce an optically active alcohol according to the present invention, added to the system after being immobilized, or can be contacted with the system via an NAD(P)H-exchangeable membrane.

Herein, the combination of an enzyme having carbonyl reductase activity and a dehydrogenase having the activity to regenerate reduced nicotinamide adenine dinucleotide (NADH) or reduced nicotinamide adenine dinucleotide phosphate (NADPH) used in the present invention is not limited, but a preferred combination includes a combination of a dehydrogenase selected from the dehydrogenases described below in (1) and an enzyme having carbonyl reductase activity selected from enzymes having carbonyl reductase activity described below in (2). More specifically, for example, when CpSADH is selected as the enzyme having carbonyl reductase activity, the dehydrogenase used in combination may be a dehydrogenase selected from any of glucose dehydrogenase, alcohol dehydrogenase, formate dehydrogenase, amino acid dehydrogenase, organic acid dehydrogenase (such as malate dehydrogenase), phosphite dehydrogenase, or hydrogenase. This is the same when an enzyme other than CpSADH is selected as the enzyme having carbonyl reductase activity. Accordingly, in the method for producing optically active alcohols of the present invention, the combination of dehydrogenase and an enzyme having carbonyl reductase activity is not limited to those described in the Examples.

### (1) Dehydrogenases

glucose dehydrogenase, alcohol dehydrogenase, formate dehydrogenase, amino acid dehydrogenase, organic acid dehydrogenase (such as malate dehydrogenase), phosphite dehydrogenase, hydrogenase

### (2) Enzymes having carbonyl reductase activity

alcohol dehydrogenase CpSADH, alcohol dehydrogenase ReSADH, carbonyl reductase ScoPAR, (2S,3S)-butanediol dehydrogenase ZraSBDH, carbonyl reductase ScGCY1, tropinone reductase HnTR1, tropinone reductase DsTR1, alcohol dehydrogenase BstADHT, alcohol dehydrogenase PfODH

Furthermore, a method of the present invention may include the step of culturing a transformant transformed with a recombinant vector comprising a polynucleotide encoding a polypeptide that can be used in the present invention. In the method of the present invention, when using live microbial cells transformed with a recombinant vector comprising a polynucleotide of the present invention, there are cases when an additional reaction system for NAD(P)H regeneration is not necessary. More specifically, by using as the host a microorganism with a high level of NAD(P)H regeneration activity, an efficient reaction can be carried out without supplementing an enzyme for NAD(P)H regeneration to the reduction reaction using the transformant. Furthermore, by simultaneously introducing to a host a DNA encoding an NAD(P)H-dependent enzyme of the present invention and a gene for glucose dehydrogenase, alcohol dehydrogenase, formate dehydrogenase, amino acid dehydrogenase, organic acid dehydrogenase (such as malate dehydrogenase), phosphite dehydrogenase, hydrogenase, or such usable for NAD(P)H regeneration, , a more efficient expression of the NAD(P)H-dependent carbonyl reductase and reduction reaction can be carried out. To introduce these two or more genes into the host, a method for transforming a host with recombinant vectors in which a plurality of vectors with different replication origins are separately introduced with genes, a method for introducing both genes into a single vector, or a method for introducing both or one of the genes into a chromosome can be used to avoid incompatibility.

When introducing a plurality of genes into a single vector, a method is used in which regions relating to the regulation of expression, such as the promoter and terminator, are linked to each gene, or alternatively the genes may be expressed as an operon containing multiple cistrons, such as the lactose operon.

For example, glucose dehydrogenases derived from *Bacillus subtilis* and *Thermoplasma acidophilum* can be used as the enzymes for NADPH regeneration. Furthermore, formate dehydrogenase derived from *Mycobacterium vaccae* can be used as the enzyme for NADH regeneration.

More specifically, for the synthesis of optically active TFIP, a coexpression plasmid introduced with both a gene of an enzyme having the ability to reduce a carbonyl, which is any one of CpSADH, ReSADH, ScoPAR, ZraSBDH, and PFODH, and a *Mycobacterium* vaccae-derived formate dehydrogenase gene (pSF-CPA4 (described in the Examples of the present invention), pSF-RED1 (described in the Examples of the present invention), pSF-SCP7 (described in the Examples of the present invention), pSF-ZRD1 (JP-A (Kokai) 2004-357639), and pSF-PFO2 (WO O1-061014) respectively) can be used. Another example is a coexpression plasmid introduced with both a gene of an enzyme having the ability to reduce a carbonyl, which is any one of ScGCY1, HnTR1, and DsTR1, and a *Bacillus subtilis*-derived glucose dehydrogenase gene (pSG-GCY1 (described in the Examples of the present invention), pSG-HNR1 (JP-A (Kokai) 2003-230398), and pSG-DSR1 (JP-A (Kokai) 2003-230398)).

The reduction reaction using an enzyme of the present invention can be carried out in water, in an organic solvent that is poorly soluble in water, for example, organic solvents such as ethyl acetate, butyl acetate, toluene, chloroform, hexane, methyl isobutyl ketone, or methyl tertiary butyl ester; a two-phase system with an aqueous medium; or a mixed system with an organic solvent soluble in water, for example, methanol, ethanol, isopropyl alcohol, acetonitrile, acetone, or dimethylsulfoxide. The reaction of the present invention can also be carried out using immobilized enzymes, membrane reactors, and the like.

Furthermore, there is no limitation on the concentration of TFAC represented by formula (1), which is the raw material of the present reaction. The reaction is usually performed at a substrate concentration of 0.01 % to 50%, preferably at 0.1 % to 20%, and more preferably at 1% to 10%. The substrate can be added either at once at the start of the reaction, or continuously or intermittently to prevent the substrate concentration in the reaction solution from becoming too high. The substrate can be placed in the reaction system in the form of TFAC represented by formula (1), but it may also be added in the form of the hydrate represented by formula (4), or as an aqueous solution of the compound represented by formula (1) or (4), or in the form of a solution in other solvents.

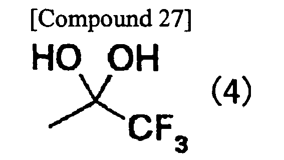

In the present invention, each of "concentration %" means "weight of the raw material or product / weight of the reaction solution (w/w)%" and "conversion rate" means "concentration of the product / ([concentration of the remaining raw material] + [concentration of the product])%". In the case of (S)-TFIP, % e.e. means "([(S)-TFIP concentration] - [(R)-TFIP concentration]) / ([(S)-TFIP concentration] + [(R)-TFIP concentration]) x 100". Similarly, in the case of (R)-TFIP, % e.e. means "([(R)-TFIP concentration] - [(S)-TFIP concentration]) / ([(R)-TFIP concentration] + [(S)-TFIP concentration]) x 100".

For reactions of the present invention, a temperature at which the enzyme can exhibit its catalytic activity can be selected. More specifically, the reaction can be carried out at a reaction temperature of 4°C to 50°C, preferably 10°C to 40°C, and more preferably 10°C to 30°C. Since the reaction substrate, TFAC, represented by formula (1) and the products, optically active TFIPs, represented by formulas (2) and (3) of the present invention all have low boiling points, the reaction is desirably carried out at low temperature. For the reaction pH, a range in which the enzyme can exhibit its catalytic activity can be selected. More specifically, the reaction can be carried out at pH 3 to 9, at pH 4 to 8, and more preferably at pH 5 to 7. Furthermore, a coenzyme, NAD⁺, NADH, NADP⁺, or NADPH, may be added as necessary to the reaction system at 0.001 mM to 100 mM, or preferably 0.01 mM to 10 mM.

To regenerate NADH and NADPH, for example, glucose is added to the reaction system when glucose dehydrogenase is used, ethanol or isopropanol is added when alcohol dehydrogenase is used, formic acid is added when formate dehydrogenase is used, amino acid is added when amino acid dehydrogenase is used, malic acid is added when malate dehydrogenase is used, glycerol is added when glycerol dehydrogenase is used, phosphorous acid is added when phosphite dehydrogenase is used, or hydrogen is added when hydrogenase is used. Such a compound may be added at a molar ratio of 0.1 to 20, or preferably 1 to 5-fold excess to the substrate ketone. On the other hand, an enzyme for NAD(P)H regeneration, such as glucose dehydrogenase, alcohol dehydrogenase, formate dehydrogenase, amino acid dehydrogenase, malate dehydrogenase, glycerol dehydrogenase, phosphite dehydrogenase, or hydrogenase, may be added at enzymatic activity 0.01 to 100 times higher, or preferably about 0.1 to 20 times higher than that of the NAD(P)H-dependent enzyme of the present invention.

Furthermore, the production method of the present invention may also include the step of collecting (S)-TFIP or (R)-RFIP. (S)-TFIP or (R)-RFIP can be collected, for example, by the method described in the Examples, but is not limited thereto.

Optically active TFIP produced by reducing TFAC according to the present invention can be purified by a suitable combination of centrifugation of bacterial cells and proteins, separation by membrane treatment, solvent extraction, distillation, drying using a drying agent, column chromatography, and such.

For example, in optically active TFIP synthesis, optically active TFIP can be obtained by distilling the reaction solution containing the microbial cells. Preferably, before the distillation procedure, a procedure to remove the bacterial cells, for example, centrifugation, membrane treatment, and such can be performed. Furthermore, for higher purity of the reaction product and especially to decrease the water content, the reaction product is dried using various types of drying agents, and the product can be purified to a higher degree by redistillation when necessary. Drying agents generally used for drying can be used.

Optically active TFIP means TFIP under a condition in which the concentration of one of the enantiomers is higher than the concentration of the other enantiomer. High optical purity sufficient for industrial use includes 90% e.e. or more, and preferably 93% e.e. or more.

All prior art references cited herein are incorporated herein by reference.

### Examples

The present invention is illustrated in detail below with reference to Examples, but is not to be construed as being limited thereto.

Herein, an LB medium refers to a medium containing 1% Bacto Tryptone, 0.5% Bacto Yeast Extract, 1% sodium chloride, and having a pH of 7.2, and an ampicillin-containing LB medium refers to a medium prepared by adding ampicillin to the LB medium with the above composition at a concentration of 50 mg/mL.

### Measurement of enzyme activities

*Escherichia coli* HB101 strain or JM 109 strain was transformed with the plasmids prepared in the Examples described below and other plasmids described in the references. The resultant transformants were cultured in ampicillin-containing LB medium overnight. 0.1 mM isopropyl-1-thio-β-D-galactopyranoside (hereinafter abbreviated as IPTG) was added to induce the expression of genes, and the cultivation was further continued for four hours. The resultant bacterial cells were collected, suspended in tris-hydrochloride or phosphate buffer solution containing 0.02% mercaptoethanol, and lysed with a closed system ultrasonic cell disrupter UCD-200TM (Cosmo Bio Co., Ltd.). The lysate was centrifuged, and the supernatant was used as a cell-free extract. Using the cell-free extract, enzyme activities were measured by the following methods.

### (Measurement of CpSADH, ReSADH, ScoPAR, and PFODH activities)

A reaction solution containing the cell-free extract, 100 mM tris-hydrochloride buffer solution (pH 9.0), a substrate, and 2.5 mM NAD⁺ was allowed to react at 30°C, and the increase in absorbance at 340 nm, resulting from NADH production, was measured. 1 U was defined as the amount of enzyme capable of catalyzing the production of 1 µmol of NADH in one minute. As substrates, 50 mM (S)-1,3-butanediol was used to measure the activity of CpSADH, 5 mM (S)-2-octanol was used to measure the activity of ReSADH and ScoPAR, and 5 mM (R)-2-octanol was used to measure the activity of PfODH.

### (Measurement of ZraSBDH, BstADHT, ScADH1, and ScADH2 activities)

A reaction solution containing the cell-free extract, 100 mM phosphate buffer solution (pH 8.0), a substrate, and 2.5 mM NAD⁺ was allowed to react at 30°C, and the increase in absorbance at 340 nm, resulting from NADH production was measured. 1 U was defined as the amount of enzyme capable of catalyzing the production of 1 µmol of NADH in one minute. As substrates, 50 mM (2S, 3S)-butanediol was used to measure the activity of ZraSBDH, and 100 mM ethanol was used to measure the activity of BstADHT, ScADH1, and ScADH2.

### (Measurement of ScGCY1, HnTR1, DsTR1, and ScGRE3 activities)

A reaction solution containing the cell-free extract, 100 mM phosphate buffer solution (pH 6.5), a substrate, and 0.2 mM NADPH was allowed to react at 30°C, and the decrease in absorbance at 340 nm, which results from the decrease of NADPH, was measured. 1 U was defined as the amount of enzyme capable of catalyzing the decrease of 1 µmol of NADPH in one minute. As substrates, 20 mM ethyl acetoacetate was used to measure the activity of ScGCY1, 4 mM tropinone was used to measure the activity of HnTR1 and DsTR1, and 100 mM xylose was used to measure the activity of ScGRE3.

### (Measurement of formate dehydrogenase activity)

A reaction solution containing the cell-free extract, 100 mM phosphate buffer solution (pH 7.0), 100 mM sodium formate, and 2.5 mM NAD⁺ was allowed to react at 30°C, and the increase in absorbance at 340 nm, resulting from NADH production, was measured. 1 U was defined as the amount of enzyme capable of catalyzing the production of 1 µmol of NADH in one minute.

### (Measurement of glucose dehydrogenase activity)

A reaction solution containing the cell-free extract, 100 mM phosphate buffer solution (pH 7.0), 100 mM D-glucose, and 2.5 mM NAD⁺ was allowed to react at 30°C, and the increase in absorbance at 340 nm, resulting from NADH production, was measured. 1 U was defined as the amount of enzyme capable of catalyzing the production of 1 µmol of NADPH in one minute.

### Analysis conditions

### (Analysis condition 1) Quantitative analysis of TFAC and TFIP

Organic layers prepared by extracting each of the reaction solutions with an equal volume of dichloromethane, or samples prepared by dissolving 10 mg of TFIP in 1 mL of acetonitrile were analyzed under the following conditions.
Column: Supelcowax 10 (Supelco) (30 m x 0.25 mm x 0.25 µm)
Column temperature: 60°C to 120°C (4°C/minute)
Inlet and detector temperature: 250°C
Detection method: hydrogen flame ionization
Carrier gas: helium (100 kPa)

As a result of the analysis under the conditions above, TFAC and TFIP were detected at retention times of 2.2 minutes and 5.6 minutes, respectively.

### (Analysis condition 2) Optical purity analysis of TFIP

Organic layers prepared by extracting each of the reaction solutions with an equal volume of dichloromethane, or samples prepared by dissolving 5 mg of TFIP in 1 mL of dichloromethane were analyzed under the following conditions.
Column: BGB-174 (BGB Analytik) (30 m x 0.25 mm x 0.25 µm)
Column temperature: 60°C to 85°C (1°C/minute) to 110°C (5°C/minute)
Inlet temperature: 180°C
Detector temperature: 200°C
Detection method: hydrogen flame ionization
Carrier gas: helium (100 kPa)

As a result of the analysis under the conditions above, (R)-TFAC and (S)-TFIP were detected at retention times of 21.8 minutes and 23.5 minutes, respectively.

### [Example 1] Construction of plasmid pSF-CPA4 coexpressing alcohol dehydrogenase CpSADH gene derived from Candida parapsilosis and formate dehydrogenase McFDH gene derived from Mycobacterium vaccae

A sense primer CPA-ATG5 (SEQ ID NO: 19) and an antisense primer CPA-TAA5 (SEQ ID NO: 20) were synthesized for cloning based on the nucleotide sequence (Accession No. E09871) described in Japanese Patent No. 3574682.
SEQ ID NO: 19 GTGGAATTCTATAATGTCAATTCCATCAAGCCAG
SEQ ID NO: 20 CTGAAGCTTATTATGGATTAAAAACAACACGACCTTCATAAGC

50 µL of a mixture containing 10 pmol each of the primers CPA-ATG5 and CPA-TAA5, 10 pmol of dNTP, 10 pmol of the plasmid pSE-CPA1 described in Biosci. Biotechnol. Biochem., 66, 481-483 (2002), and 1.25 U of Pfu Turbo DNA polymerase (STRATAGENE) was subjected to 30 PCR cycles of denaturation at 95°C for 30 seconds, annealing at 50°C for 1 minute, and extension at 72°C for 2 minutes 30 seconds using GeneAmp PCR System 2400, thereby obtaining a specific amplification product.

The amplified product was double-digested with restriction enzymes NcoI and XbaI, and then electrophoresed in an agarose gel. The band of interest was cut out, and then purified using GFX PCR DNA and Gel Band Purification Kit (Pharmacia).

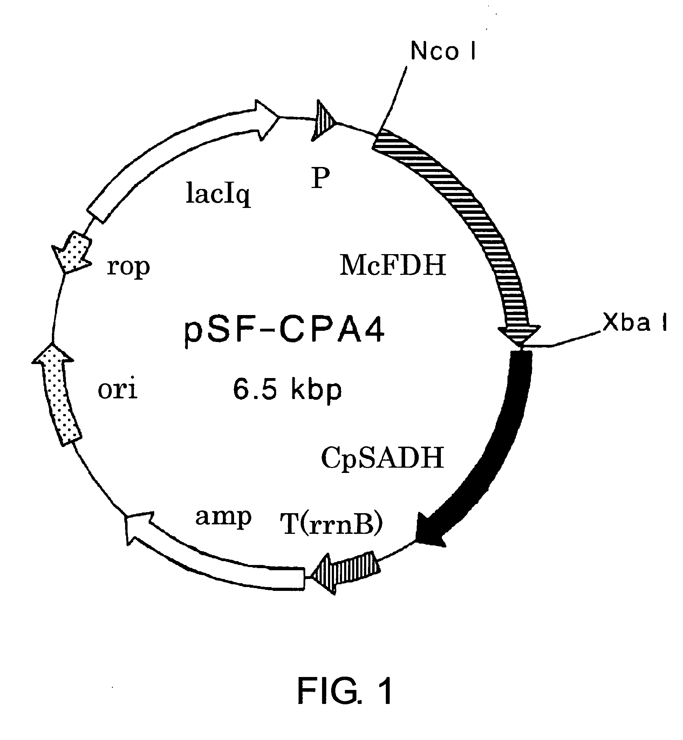

The resultant PCR-amplified DNA fragments after digestion with the restriction enzymes were ligated with a vector pSE-MF26 (JP-A (Kokai) 2003-199595), which had been double-digested with restriction enzymes NcoI and XbaI using TAKARA Ligation Kit. *Escherichia coli* JM109 strain was transformed with the ligated DNA. The transformant was grown in ampicillin-containing LB medium, and then a plasmid was purified from the transformant using FlexiPrep Kit (Pharmacia). The nucleotide sequence of the DNA insert of the purified plasmid was analyzed, and the determined nucleotide sequence and its amino acid sequence are indicated by SEQ ID NO: 1 and SEQ ID NO: 9, respectively. The plasmid obtained was named pSF-CPA4 (Fig. 1).

### [Example 2] Enzyme activity of the transformant transformed with plasmid coexpressing alcohol dehydrogenase CpSADH derived from Candida parapsilosis and formate dehydrogenase McFDH derived from Mycobacterium vaccae

A cell-free extract was prepared according to the above-described method using the *Escherichia coli* HB101 strain transformed with pSF-CPA4 obtained in Example 1. The enzyme activity was measured according to the above-described method. The specific activities of CpSADH and McFDH were 5.96 U/mg protein and 0.474 U/mg protein, respectively.

### [Example 3] Preparation of chromosomal DNA from Rhodococcus erythropolis

*Rhodococcus erythropolis* DSM 743 strain was cultured in a broth medium, and bacterial cells were prepared. Preparation of chromosomal DNA from the bacterial cells was performed by the method described in Nucleic Acids Res., 8, 4321 (1980).

### [Example 4] Cloning of alcohol dehydrogenase ReSADH derived from Rhodococcus erythropolis

A sense primer RE-ATG1 (SEQ ID NO: 21) and an antisense primer RE-TAA1 (SEQ ID NO: 22) were synthesized for cloning based on the nucleotide sequence (Accession No. AY161280) described in Appl. Microbiol. Biotechnol., 62, 380-386 (2003).
SEQ ID NO: 21 CACGAATTCTATCATGAAAGCAATCCAGTACACG
SEQ ID NO: 22 TCGAAGCTTCTAGATTAAAGACCAGGGACCACAAC

50 µL of a mixture containing 10 pmol each of the primers RE-ATG1 and RE-TAA1, 10 nmol of dNTP, 50 ng of the *Rhodococcus erythropolis*-derived chromosome prepared in Example 3, and 1.25 U of Pfu Turbo DNA polymerase (STRATAGENE) was subjected to 30 PCR cycles of denaturation at 95°C for 30 seconds, annealing at 50°C for 1 minute, and extension at 72°C for 2 minutes 30 seconds using GeneAmp PCR System 2400, thereby obtaining a specific amplification product.

The amplified product was double-digested with restriction enzymes EcoRI and HindIII, and then electrophoresed in an agarose gel. The band of interest was cut out, and then purified using GFX PCR DNA and Gel Band Purification Kit (Pharmacia).

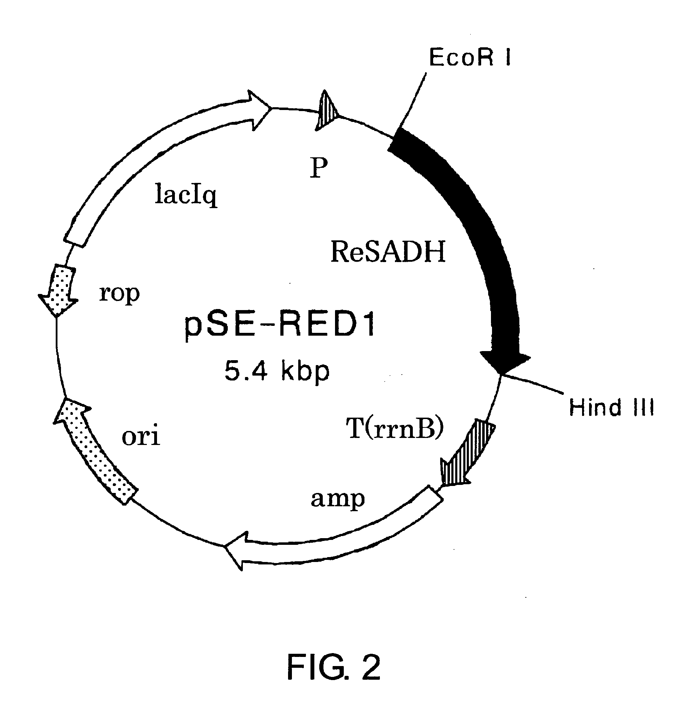

The resultant PCR-amplified DNA fragments after digestion with the restriction enzymes were ligated with a vector pSE420D (JP-A (Kokai) 2000-189170), which had been double-digested with restriction enzymes EcoRI and Hind III using TAKARA Ligation Kit. *Escherichia coli* JM109 strain was transformed with the ligated DNA. The transformant was grown in an ampicillin-containing LB medium, and then a plasmid was purified from the transformant using FlexiPrep Kit (Pharmacia). The nucleotide sequence of the DNA insert of the purified plasmid was analyzed and compared with the nucleotide sequence described in the reference; except for the primer region, substitutions of four bases and one amino acid were found. The determined nucleotide sequence and its amino acid sequence are indicated by SEQ ID NO: 2 and SEQ ID NO: 10, respectively. The plasmid obtained was named pSE-RED1 (Fig. 2).

### [Example 5] Construction of plasmid pSF-RED1 coexpressing alcohol dehydrogenase ReSADH gene derived from Rhodococcus erythropolis and formate dehydrogenase McFDH gene derived from Mycobacterium vaccae

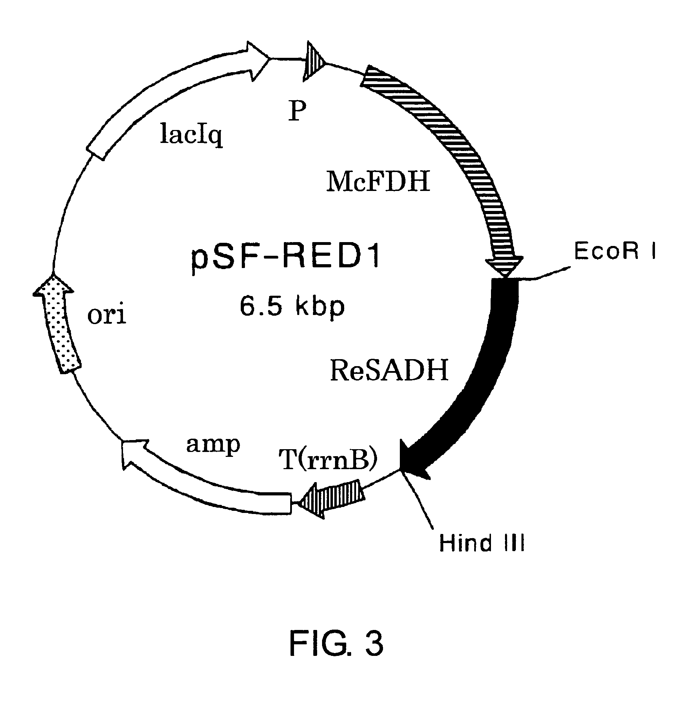

pSE-RED1 obtained in Example 4 was double-digested with restriction enzymes EcoRI and HindIII, and the resultant DNA fragments were purified. Further, plasmid pSE-MF26 (JP-A (Kokai) 2003-199595) containing a formate dehydrogenase gene derived from *Mycobacterium vaccae* was double-digested with restriction enzymes EcoRI and HindIII, and ligated with the DNA fragment cut out from pSL-RED1 using TAKARA Ligation Kit. *Escherichia coli* JM109 strain was transformed with the ligated DNA. The transformant was grown in an ampicillin-containing LB medium, and then a plasmid was purified from the resultant transformant using FlexiPrep Kit (Pharmacia) to obtain the plasmid pSF-RED1 (Fig. 3) capable of coexpressing ReSADH and formate dehydrogenase McFDH.

### [Example 6] Enzyme activity of the transformant transformed with a plasmid coexpressing alcohol dehydrogenase ReSADH derived from Rhodococcus erythropolis and formate dehydrogenase McFDH derived from Mycobacterium vaccae

A cell-free extract was prepared according to the above-described method using the *Escherichia coli* HB101 strain transformed with pSF-RED1 obtained in Example 5. The enzyme activity was measured according to the above-described method. The specific activities of ReSADH and McFDH were 8.49 U/mg protein and 1.52 U/mg protein, respectively.

### [Example 7] Cloning of carbonyl reductase ScoPAR derived from Streptomyces coelicolor

DNA was synthesized based on the amino acid sequence of SEQ ID NO: 11. The sequence of the synthetic DNA is indicated by SEQ ID NO: 3. The resultant sequence was double-digested with restriction enzymes EcoRI and HindIII, and then purified.

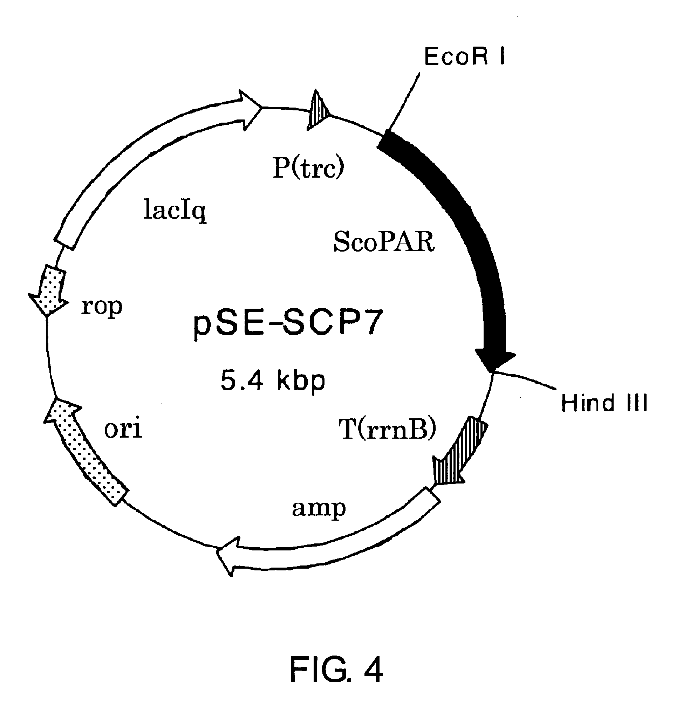

The resultant synthetic DNA fragments after digestion with the restriction enzymes were ligated with the vector pSE420D (JP-A (Kokai) 2000-189170), which had been double-digested with restriction enzymes EcoRI and HindIII, using TAKARA Ligation Kit. *Escherichia coli* JM109 strain was transformed with the ligated DNA. The transformant was grown in an ampicillin-containing LB medium, and a plasmid was purified from the resultant transformant using FlexiPrep Kit (Pharmacia). The nucleotide sequence of the DNA insert of the purified plasmid was analyzed, and found to be consistent with the nucleotide sequence of SEQ ID NO: 11. The plasmid obtained was named pSE-SCP7 (Fig. 4).

### [Example 8] Construction of plasmid pSF-SCP7 coexpressing carbonyl reductase ScoPAR derived from Streptomyces coelicolor and formate dehydrogenase McFDH gene derived from Mycobacterium vaccae

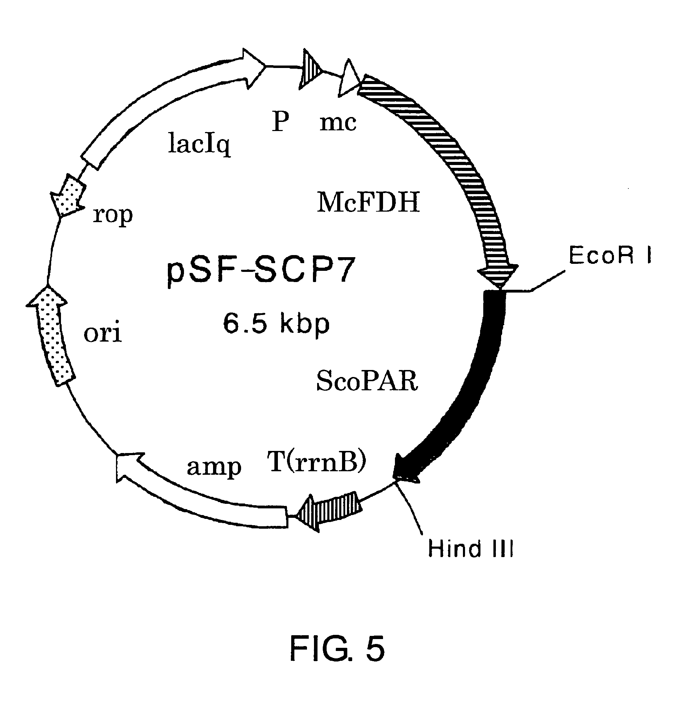

pSE-SCP7 obtained in Example 7 was double-digested with restriction enzymes EcoRI and HindIII, and the resultant DNA fragments were purified. Further, the plasmid pSU-MF26 (Japanese Patent Application No. 2005-169919) containing a formate dehydrogenase McFDH gene derived from *Mycobacterium vaccae* was double-digested with restriction enzymes EcoRI and HindIII, and ligated with DNA fragments cut out from the pSL-SCP7. *Escherichia coli* JM109 strain was transformed with the ligated DNA. The transformant was grown in an ampicillin-containing LB medium, and a plasmid was purified from the resultant transformant using FlexiPrep Kit (Pharmacia) to obtain the plasmid pSF-SCP7 (Fig. 5) capable of coexpressing ScoPAR and McFDH.

### [Example 9] Enzyme activity of the transformant transformed with a plasmid coexpressing carbonyl reductase ScoPAR derived from Streptomyces coelicolor and formate dehydrogenase McFDH derived from Mycobacterium vaccae

A cell-free extract was prepared according to the above-described method using the *Escherichia coli* HB101 strain transformed with pSF-SCP7 obtained in Example 8. The enzyme activity was measured according to the above-described method. The specific activities of ScoPAR and McFDH were 0.67 mU/mg protein and 0.25 U/mg protein, respectively.

### [Example 10] Preparation of chromosomal DNA from Saccharomyces cerevisiae

*Saccharomyces cerevisiae* was cultured in a YEPD medium, and bacterial cells were prepared. Preparation of chromosomal DNA from the bacterial cells was performed by the method described in Meth. Cell Biol., 29, 39 (1975).

### [Example 11] Cloning of carbonyl reductase ScGCY1 derived from Saccharomyces cerevisiae

A sense primer GCY1-ATG1 (SEQ ID NO: 23) and an antisense primer GCY1-TAA1 (SEQ ID NO: 24) were synthesized for cloning based on the nucleotide sequence (Accession No. X13228) of the carbonyl reductase gene derived from *Saccharomyces cerevisiae* described in FEBS Lett., 238, 123-128 (1988).
SEQ ID NO: 23 GAGCCATGGCACCTGCTACTTTACATGATTCTACGAA
SEQ ID NO: 24 GAGCTTAAGTCTAGATTATTTGAATACTTCGAAAGGAGACCA

Using the chromosomal DNA obtained in Example 10, which had been purified from *Saccharomyces cerevisiae,* as the template, PCR was carried out in the same manner as in Example 4 using the primers GCY1-ATG1 and GCY1-TAA1.

The amplified product was double-digested with restriction enzymes NcoI and XbaI, and then electrophoresed in an agarose gel. The band of interest was cut out, and then purified using GFX PCR DNA and Gel Band Purification Kit (Pharmacia).

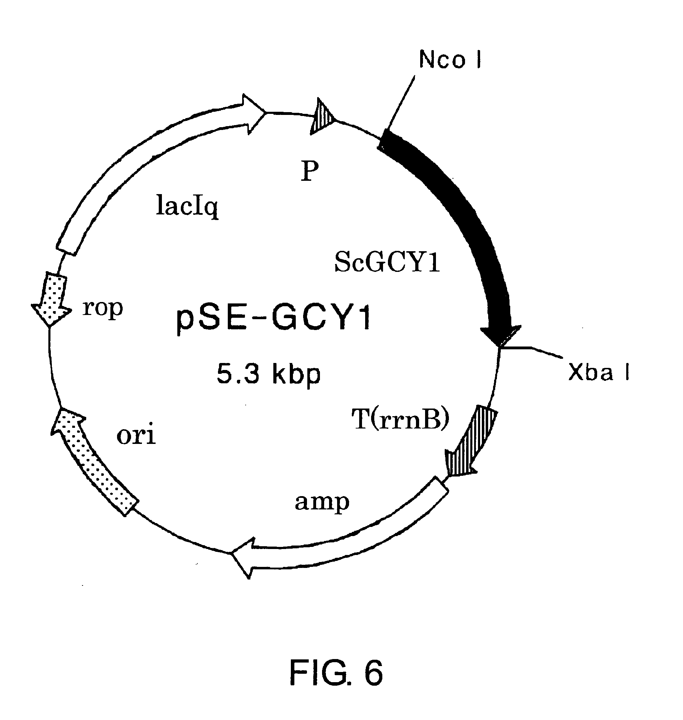

The resultant PCR-amplified DNA fragments after digestion with the restriction - enzymes were ligated with a vector pSE420D (JP-A (Kokai) 2000-189170), which had been double-digested with restriction enzymes NcoI and XbaI. *Escherichia coli* JM109 strain was transformed with the ligated DNA. The transformant was grown in an ampicillin-containing LB medium, and a plasmid was purified from the resultant transformant using FlexiPrep Kit (Pharmacia). The nucleotide sequence of the DNA insert of the purified plasmid was analyzed, and found to be consistent with the nucleotide sequence described in the reference, except for the primer region. The determined nucleotide sequence and its amino acid sequence are indicated by SEQ ID NO: 5 and SEQ ID NO: 13, respectively. The plasmid obtained was named pSE-GCY1 (Fig. 6).

### [Example 12] Construction of plasmid pSG-GCY1xpressing carbonyl reductase ScGCY1 derived from Saccharomyces cerevisiae and glucose dehydrogenase BsGDH gene derived from Bacillus subtilis

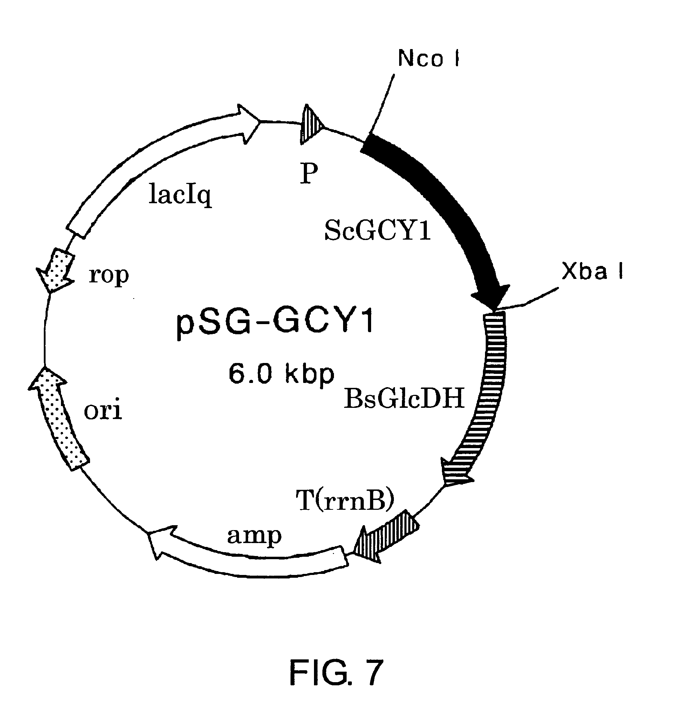

pSE-GCY1 obtained in Example 11 was double-digested with restriction enzymes NcoI and XbaI, and the resultant DNA fragments were purified. Further, the plasmid pSE-BSG1 (JP-A (Kokai) 2000-189170) containing glucose dehydrogenase BsGDH gene derived from *Bacillus subtilis* was double-digested with restriction enzymes NcoI and XbaI, and then ligated with DNA fragments cut out from the pSL-GCY1. *Escherichia coli* JM109 strain was transformed with the ligated DNA. The transformant was grown in an ampicillin-containing LB medium, and a plasmid was purified from the resultant transformant using FlexiPrep Kit (Pharmacia) to obtain a plasmid pSG-GCY1 capable of coexpressing ScGCY1 and BsGDH (Fig. 7).

### [Example 13] Enzyme activity of the transformant transformed with a plasmid coexpressing carbonyl reductase ScGCY1 derived from Saccharomyces cerevisiae and glucose dehydrogenase BsGDH derived from Bacillus subtilis

A cell-free extract was prepared according to the above-described method using the *Escherichia coli* HB101 strain transformed with pSF-GCY1 obtained in Example 12. The enzyme activity was measured according to the above-described method. The specific activities of ScGCY1 and BsGDH were 0.27 U/mg protein and 3.72 U/mg protein, respectively.

### [Example 14] Cloning of alcohol dehydrogenase BstADHT derived from Geobacillus stearothermophilus

A sense primer BSAT-AT1 (SEQ ID NO: 25) and an antisense primer BSAT-TA1 (SEQ ID NO: 26) were synthesized for cloning based on the nucleotide sequence (Accession No. D90421) of an alcohol dehydrogenase gene derived from *Geobacillus stearothermophilus* described in J. Bacteriol., 174, 1397-1402 (1992).
SEQ ID NO: 25 GAGGAATTCAATCATGAAAGCTGCAGTTGTG
SEQ ID NO: 26 GTCAAGCTTCTAGATTAATCTACTTTTAACACGACGC

Using the plasmid pTBAD40 as the template, PCR was carried out in the same manner as in Example 4 using the primers BSAT-AT1 and BSAT-TA1.

The amplified product was double-digested with restriction enzymes BspHI and XbaI, and electrophoresed in an agarose gel. The band of interest was cut out, and then purified using GFX PCR DNA and Gel Band Purification Kit (Pharmacia).

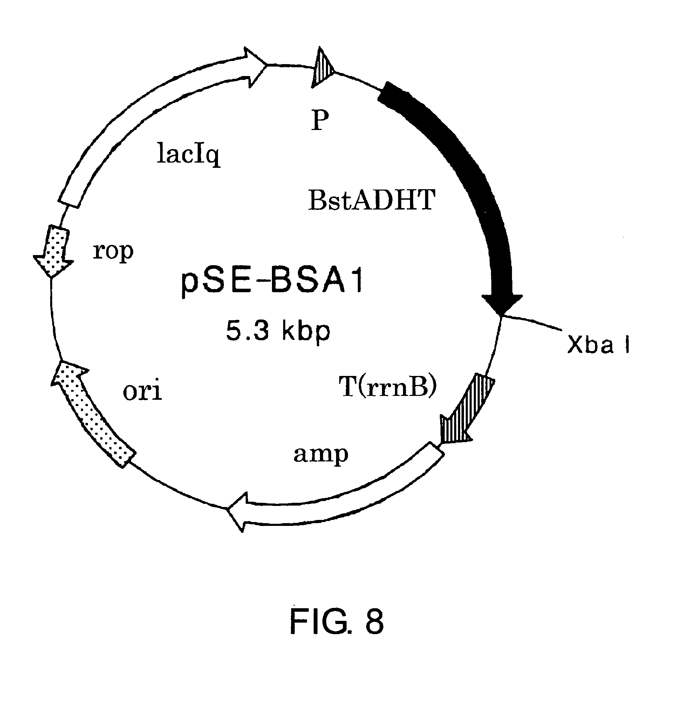

The resultant PCR-amplified DNA fragments after digestion with the restriction enzymes were ligated with a vector pSE420D (JP-A (Kokai) 2000-189170), which had been double-digested with restriction enzymes NcoI and XbaI. *Escherichia coli* JM109 strain was transformed with the ligated DNA. The transformant was grown in an ampicillin-containing LB medium, and a plasmid was purified from the resultant transformant using FlexiPrep Kit (Pharmacia). The nucleotide sequence of the DNA insert of the purified plasmid was analyzed, and found to be consistent with the nucleotide sequence described in the reference, except for the primer region. The determined nucleotide sequence and its amino acid sequence are indicated by SEQ ID NO: 8 and SEQ ID NO: 16, respectively. The plasmid obtained was named pSE-BSA1 (Fig. 8).

### [Example 15] Enzyme activity of the transformant transformed with a plasmid expressing alcohol dehydrogenase BstADHT derived from Geobacillus stearothermophilus

A cell-free extract was prepared according to the above-described method using the *Escherichia coli* HB101 strain transformed with the pSE-BSA1 obtained in Example 1. The enzyme activity was measured according to the above-described method. The specific activity of BstADHT was 1.08 U/mg protein.

### [Reference Example 1) Cloning of alcohol dehydrogenase-I, SCADH1 derived from Saccharomyces cerevisiae

S sense primer ScADH1-A1 (SEQ ID NO: 27) and an antisense primer ScADH1-T1 (SEQ ID NO: 28) were synthesized for cloning based on the nucleotide sequence (Accession No. M38456) of an alcohol dehydrogenase-I gene derived from *Saccharomyces cerevisiae* described in Basic Life Sci., 19, 335-361 (1982).
SEQ ID NO: 27 GTCGAATTCATACATGTCTATCCCAGAAACTCAAAAAGG
SEQ ID NO: 28 CTGCTTAAGTCTAGATTATTTAGAAGTGTCAACAACGTAACGACCAA

Using the chromosomal DNA obtained in Example 10, which had been purified from *Saccharomyces cerevisiae,* as the template, PCR was carried out in the same manner as in Example 4 using the primers ScADH1-A1 and ScADH1-T1.

The amplified product was double-digested with restriction enzymes EcoRI and AflII, and electrophoresed in an agarose gel. The band of interest was cut out, and then purified using GFX PCR DNA and Gel Band Purification Kit (Pharmacia).

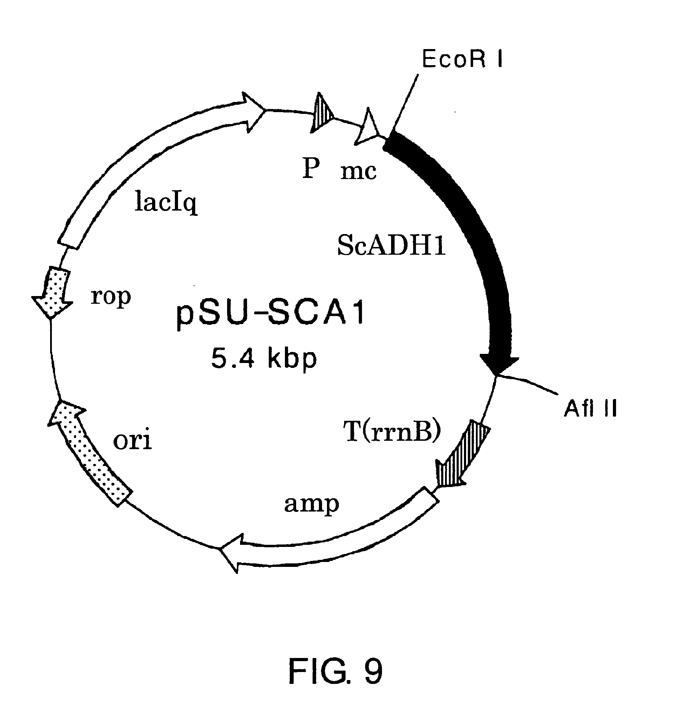

The resultant PCR-amplified DNA fragments after digestion with the restriction enzymes were ligated with the vector pSE420U (Japanese Patent Application No. 2005-169919), which had been double-digested with restriction enzymes EcoRI and Af1II. *Escherichia coli* JM109 strain was transformed with the ligated DNA. The transformant was grown in an ampicillin-containing LB medium, and a plasmid was purified from the resultant transformant using FlexiPrep Kit (Pharmacia). The nucleotide sequence of the DNA insert of the purified plasmid was analyzed and compared with the nucleotide sequence described in the reference; except for the primer region, substitutions of ten bases and one amino acid were found. The determined nucleotide sequence and its amino acid sequence are indicated by SEQ ID NO: 29 and SEQ ID NO: 30, respectively. The plasmid obtained was named pSU-SCA1 (Fig. 9).

### [Reference Example 2] Enzyme activity of the transformant transformed with a plasmid expressing alcohol dehydrogenase-I, ScADH1 derived from Saccharomyces cerevisiae

A cell-free extract was prepared according to the above-described method using the *Escherichia coli* JM109 strain transformed with the pSU-SCA1 obtained in Reference Example

### 1. The enzyme activity was measured according to the above-described method. The specific activity of ScADH1 was 4.92 U/mg protein.

### [Reference Example 3] Cloning of alcohol dehydrogenase-II, ScADH2 derived from Saccharomyces cerevisiae

A sense primer ScADH2-A1 (SEQ ID NO: 31) and an antisense primer ScADH2-T1 (SEQ ID NO: 32) were synthesized for cloning based on the nucleotide sequence (Accession No. J01314, M13475) of an alcohol dehydrogenase-II gene derived from *Saccharomyces cerevisiae* described in J. Biol. Chem., 258, 2674-2682 (1983).
SEQ ID NO: 31 GTCGAATTCATACATGTCTATTCCAGAAACTCAAAAAGC
SEQ ID NO: 32 GCACTTAAGTCTAGATTATTTAGAAGTGTCAACAACGTAACGACCAG

Using as the template the chromosomal DNA purified from *Saccharomyces cerevisiae* in Example 10, PCR was carried out in the same manner as in Example 4 using the primers ScADH2-A1 and ScADH2-T1.

The amplified product was double-digested with restriction enzymes EcoRI and AflII, and electrophoresed in an agarose gel. The band of interest was cut out, and then purified using GFX PCR DNA and Gel Band Purification Kit (Pharmacia).

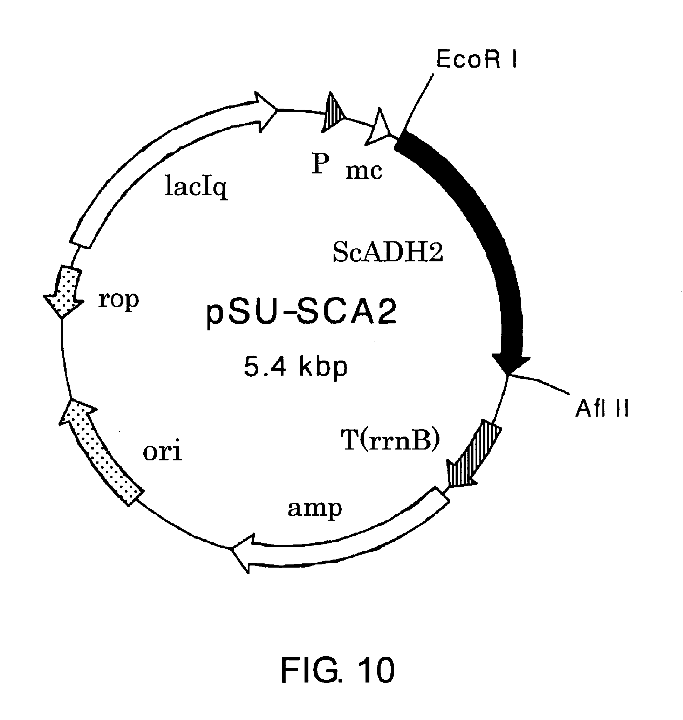

The resultant PCR-amplified DNA fragments after digestion with the restriction enzymes were ligated with the vector pSE420U (Japanese Patent Application No. 2005-169919), which had been double-digested with restriction enzymes EcoRI and AflII. *Escherichia coli* JM 109 strain was transformed with the ligated DNA. The transformant was grown in an ampicillin-containing LB medium and a plasmid was purified from the resultant transformant using FlexiPrep Kit (Pharmacia). The nucleotide sequence of the DNA insert of the purified plasmid was analyzed and compared with the nucleotide sequence described in the reference; except for the primer region, substitution of one base was found. The determined nucleotide sequence and its amino acid sequence are indicated by SEQ ID NO: 33 and SEQ ID NO: 34, respectively. The plasmid obtained was named pSU-SCA2 (Fig. 10).

### [Reference Example 4] Enzyme activity of the transformant transformed with a plasmid expressing alcohol dehydrogenase-II, ScADH2 derived from Saccharomyces cerevisiae

A cell-free extract was prepared according to the above-described method using the *Escherichia coli* JM109 strain transformed with the pSU-SCA2 obtained in Reference Example 3. The enzyme activity was measured according to the above-described method. The specific activity of ScADH2 was 32.3 U/mg protein.

### [Reference Example 5] Cloning of carbonyl reductase ScGRE3 derived from Saccharomyces cerevisiae

A sense primer GRE3-ATG1 (SEQ ID NO: 35) and an antisense primer GRE3-TAA1 (SEQ ID NO: 36) were synthesized for cloning, based on the nucleotide sequence (Accession No. U00059) of a carbonyl reductase gene derived from *Saccharomyces cerevisiae* described in Appl. Environ. Microbiol., 61, 1580-1585 (1995).
SEQ ID NO: 35 GAGTCATGAGTTCACTGGTTACTCTTAATAACGGTC
SEQ ID NO: 36 GACGAATTCCTCTAGATTATGCAAAAGTGGGGAATTTACCATC

Using the chromosomal DNA obtained in Example 10, which had been purified from *Saccharomyces cerevisiae,* as the template, PCR was carried out in the same manner as in Example 4 using the primers GRE3-ATG1 and GRE3-TAA1.

The amplified product was double-digested with restriction enzymes BspHI and EcoRI, and then electrophoresed in an agarose gel. The band of interest was cut out, and then purified using GFX PCR DNA and Gel Band Purification Kit (Pharmacia).

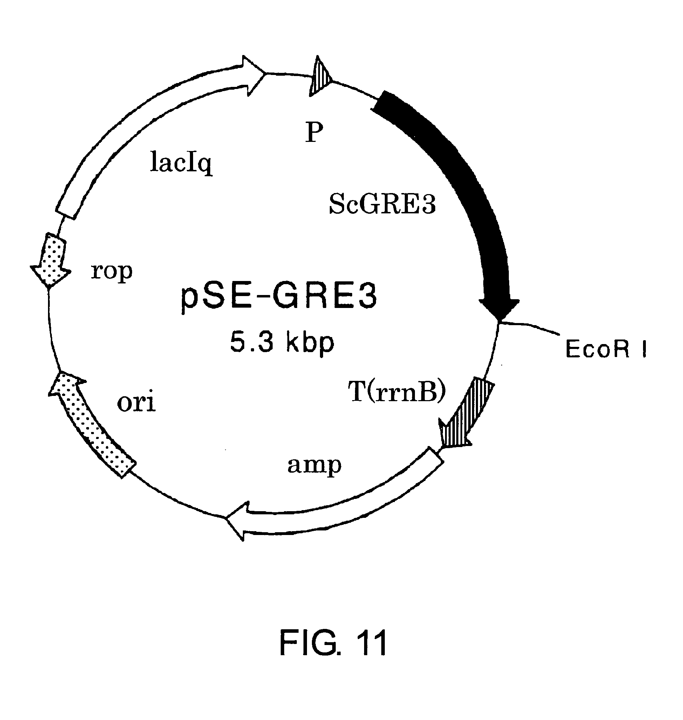

The resultant PCR-amplified DNA fragments after digestion with the restriction enzymes were ligated with the vector pSE420D (JP-A (Kokai) 2000-189170), which had been double-digested with restriction enzymes NcoI and EcoRI. *Escherichia coli* JM109 strain was transformed with the ligated DNA. The transformant was grown in an ampicillin-containing LB medium, and a plasmid was purified from the resultant transformant using FlexiPrep Kit (Pharmacia). The nucleotide sequence of the DNA insert of the purified plasmid was analyzed, and found to be consistent with the nucleotide sequence described in the reference. The determined nucleotide sequence and its amino acid sequence are indicated by SEQ ID NO: 37 and SEQ ID NO: 38, respectively. The plasmid obtained was named pSE-GRE3 (Fig. 11).

### [Reference Example 6] Enzyme activity of the transformant transformed with a plasmid expressing carbonyl reductase ScGRE3 derived from Saccharomyces cerevisiae

A cell-free extract was prepared according to the above-described method using the *Escherichia coli* JM109 strain transformed with the pSE-GRE3 obtained in Example 20. The enzyme activity was measured according to the above-described method. The specific activity of ScGRE3 was 206 mU/mg protein.

### [Example 16] Preparation of TFAC hydrate

40 mL of 100 mM phosphate buffer solution (pH 7.4) was placed in 100-mL volumetric flask and cooled on an ice bath. To the flask 1,1,1-trifluoroacetone (TFAC) was added slowly and dissolved in the solution to make 100 mL, thereby obtaining a TFAC hydrate containing TFAC at a concentration of 944 g/L.

### [Example 17] Production of optically active TFIP using TFAC as a substrate

*Escherichia coli* HB101 strain was transformed with each of plasmids pSF-CPA4 (described in Example 1 herein), pSF-RED1 (described in Example 5 herein), pSF-SCP7 (described in Example 8 herein), pSF-ZRD1 (JP-A (Kokai) 2004-357639), and pSF-PFO2 (WO 01-061014), and the resultant transformants were cultured in a 2 x YT medium (2% Bacto Tryptone, 1% Bacto Yeast Extract, 1% sodium chloride, and pH 7.2) overnight at 33°C. 0.1 mM IPTG was added to the medium to induce expression of each of the enzyme genes, and then the bacterial cells were collected to obtain *Escherichia coli* capable of expressing each of the enzyme genes.

The TFAC hydrate obtained in Example 16, 100 mM phosphate buffer solution (pH 6.5), two equivalents of sodium formate relative to TFAC, and bacterial cells collected from 10 g of the culture medium were placed in a reaction vessel to prepare a total of 10 g of reaction solution containing TFAC as the substrate at a final concentration of 2% (178 mM). The reaction solution was rotary shaken overnight at 20°C. 0.8 mL portions were sampled from the reaction solution, and extracted with 0.8 mL of dichloromethane. The organic layers were analyzed under the analysis conditions 1 and 2 described below to quantify TFIP and analyze its optical purity. The results are shown in Table 1.

**[Table 1]**

| Example, Comparative Example | Plasmid | Enzyme | Yield % | Optical Purity % e.e. | Absolute Configuration |
|---|---|---|---|---|---|
| Example 17 | pSF-CPA4 | CpSADH -McFDH | 67.8 | 99.1 | S |
| Example 17 | pSF-RED1 | ReSADH -McFDH | 63.0 | 98.7 | S |
| Example 17 | pSF-SCP7 | ScoPAR -McFDH | 49.4 | 98.5 | S |
| Example 17 | pSF-ZRD1 | ZraSBDH -McFDH | 64.3 | 97.8 | S |
| Example 18 | pSG-GCY1 | ScGCY1 -BsGDH | 7.0 | 93.0 | S |
| Example 18 | pSG-HNR1 | HnTR1 -BsGDH | 11.4 | 94.6 | S |
| Example 18 | pSG-DSR1 | DsTR1 -BsGDH | 20.6 | 93.5 | S |
| Example 19 | pSE-BSA1 | BstADHT -Amano2 | 8.7 | 93.9 | S |
| Example 17 | pSF-PFO2 | PfODH -McFDH | 73.4 | 100.0 | R |
| Comparative Example 1 | pUC-SCA1 | ScADH1 -Amano2 | 5.2 | 82.3 | S |
| Comparative Example 1 | pUC-SCA2 | ScADH2 -Amano2 | 8.9 | 58.0 | S |
| Comparative Example 1 | pSE-GRE3 | ScGRE3 -Amano2 | 8.3 | 69.6 | S |

### [Example 18] Production of optically active TFIP using TFAC as a substrate

*Escherichia coli* HB101 strain was transformed with each of the plasmids pSG-GCY1 (described in Example 12 herein), pSG-HNR1(JP-A (Kokai) 2003-230398), and pSG-DSR1 (JP-A (Kokai) 2003-230398), and the resultant transformants were cultured in a 2 x YT medium (2% Bacto Tryptone, 1% Bacto Yeast Extract, 1% sodium chloride, and pH 7.2) overnight at 33°C. 0.1 mM IPTG was added to the medium to induce expression of each of the enzyme genes, and then the bacterial cells were collected to obtain *Escherichia coli* expressing each of the enzyme genes.

The TFAC hydrate obtained in Example 16, 100 mM phosphate buffer solution (pH 6.5), two equivalents of D-glucose relative to TFAC, and bacterial cells collected from 10 g of the culture medium were placed in a reaction vessel to prepare a total of 10 g of reaction solution containing TFAC as the substrate at a final concentration of 2% (178 mM). The reaction solution was rotary shaken overnight at 20°C. 0.8 mL portions were sampled from the reaction solution, and extracted with 0.8 mL of dichloromethane. The organic layers were analyzed under the analysis conditions 1 and 2 described below to quantify TFIP and analyze its optical purity. The results are shown in Table 1.

### [Example 19] Production of optically active TFIP using TFAC as a substrate

*Escherichia coli* HB101 strain was transformed with a plasmid pSE-BSA1 (described in Example 14 herein), and the resultant transformant was cultured in a 2 x YT medium (2% Bacto Tryptone, 1% Bacto Yeast Extract, 1% sodium chloride, and pH 7.2) overnight at 33°C. 0.1 mM IPTG was added to the medium to induce expression of each of the enzyme genes, and then the bacterial cells were collected to obtain *Escherichia coli* expressing each of the enzyme genes.

The TFAC hydrate obtained in Example 16, 100 mM phosphate buffer solution (pH 6.5), two equivalents of D-glucose relative to TFAC, bacterial cells collected from 10 g of the culture medium, and 5 U glucose dehydrogenase (Amano-2, Amano Enzyme Inc.) were placed in a reaction vessel to prepare a total of 10 g of reaction solution containing TFAC as the substrate at a final concentration of 2% (178 mM). The reaction solution was rotary shaken overnight at 20°C. 0.8 mL portions were sampled from the reaction solution, and extracted with 0.8 mL of dichloromethane. The organic layers were analyzed under the analysis conditions 1 and 2 described below to quantify TFIP and analyze its optical purity. The results are shown in Table 1.

### [Example 20] Production of (S)-TFIP using TFAC as a substrate

*Escherichia coli* HB101 strain was transformed with a plasmid pSF-CPA4 (described in Example 1 herein), and the resultant transformant was cultured in a 2 x YT medium (2% Bacto Tryptone, 1% Bacto Yeast Extract, 1% sodium chloride, and pH 7.2) overnight at 33°C. 0.1 mM IPTG was added to the medium to induce expression of each of the enzyme genes, and then the bacterial cells were collected to obtain *Escherichia coli* coexpressing CpSADH and McFDH.

The TFAC hydrate obtained in Example 20, 100 mM phosphate buffer solution (pH 6.0), 803 mM sodium formate, and bacterial cells collected from 267 g of the culture medium were placed in a reaction vessel to prepare a total of 400 g of reaction solution containing TFAC as the substrate at a final concentration of 6% (535 mM). The reaction solution was allowed to react at 20°C under stirring with the pH controlled at 6.0 with 40% sulfuric acid. After a lapse of 26 hours, 0.8 mL portions were sampled from the reaction solution, and extracted with 0.8 mL of dichloromethane. The organic layers were analyzed under the analysis conditions 1 and 2 described below to quantify TFIP and analyze its optical purity. The conversion rate was 99% and optical purity was 99.8% e.e. (S).

### [Example 21] Purification of (S)-TFIP

Further, 1230 g of the reaction solution obtained in Example 20 was centrifuged thereby removing bacterial cells. The resultant supernatant was distilled, and the distillate at a vapor temperature of 77-79°C was collected as the main fraction in a yield of 60.17 g. To 59.02 g of the main fraction, 11.8 mL of a saturated calcium chloride aqueous solution was added, and the mixture was stirred for one hour at room temperature. Thereafter, the mixture was allowed to stand to separate the organic layer. The organic layer was distilled again, and the distillate at a vapor temperature of 72-74°C was collected as the main fraction in a yield of 48.63 g. The main fraction was analyzed under the analysis conditions 1 and 2; the TFIP purity was 97.3% and optical purity was 99.7% e.e. (S).

### [Comparative Example 1] Production of (S)-TFIP using TFAC as a substrate

*Escherichia coli* HB101 strain was transformed with plasmids pSU-SCA1 (described in Reference Example 1 herein), pSU-SCA2 (described in Reference Example 3 herein), and pSE-GRE3 (described in Reference Example 5 herein), and the resultant transformants were cultured in a 2 x YT medium (2% Bacto Tryptone, 1% Bacto Yeast Extract, 1% sodium chloride, and pH 7.2) overnight at 33°C. 0.1 mM IPTG was added to the medium to induce expression of each of the enzyme genes, and then the bacterial cells were collected to obtain *Escherichia coli* expressing each of the enzyme genes.

The TFAC hydrate obtained in Example 20, 100 mM phosphate buffer solution (pH 6.5), two equivalents of D-glucose relative to TFAC, bacterial cells collected from 10 g of the culture medium, and 5 U glucose dehydrogenase (Amano-2, Amano Enzyme Inc.) were placed in a reaction vessel to prepare a total of 10 g of reaction solution containing TFAC as the substrate at a final concentration of 2% (178 mM). The reaction solution was rotary shaken overnight at 20°C. 0.8 mL portions were sampled from the reaction mixture, and extracted with 0.8 mL of dichloromethane. The organic layers were analyzed under the analysis conditions 1 and 2 described below to quantify TFIP and analyze its optical purity. The results are shown in Table 1.

### [Comparative Example 2] Influence of reaction pH on optical purity

Reaction was carried out in the same manner as in Example 20, except that the final concentration of the substrate added to the reaction solution was 5% (446 mM), the pH of the phosphate buffer solutions was 6.5, and the pH during the reaction was controlled at 6.5. The resultant optical purity was 99.1-99.2% e.e. (S).

### Industrial Applicability

The present invention provides methods for enzymatically producing optically active (S)-1,1,1-trifluoro-2-propanol and (R)-1,1,1-trifluoro-2-propanol. Both (S)-1,1,1-trifluoro-2-propanol and (R)-1,1,1-trifluoro-2-propanol produced by the method of the present invention have high optical purity. That is, compared to a conventional method that uses an optical resolution agent, higher optical purity can be accomplished more easily and at lower cost.

Furthermore, according to the present invention, the compound of interest can be obtained efficiently using a large amount of raw material. More specifically, the present invention enables, for example, convenient and economical industrial production of (S)-1,1,1-trifluoro-2-propanol and (R)-1,1,1-trifluoro-2-propanol. That is, substances of the present invention with enzymatic activities are not easily inhibited by large amount of substrate and also by the reaction products produced from the substrate. Therefore, the present invention enables use of a larger amount of raw material to efficiently obtain the compound of interest.

(S)-1,1,1-trifluoro-2-propanol and (R)-1,1,1-trifluoro-2-propanol produced by the method of the present invention will be useful as optically active raw materials for various types of pharmaceutical products, liquid crystalline materials, and such.

## Claims

1. A method for producing (S)-1,1,1-trifluoro-2-propanol represented by formula (2), 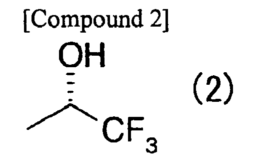 which comprises the step of reacting a protein encoded by the polynucleotide of any one of the following (a) to (e), a microorganism or a transformant strain that functionally expresses said protein, or a processed material thereof, with 1,1,1-trifluoroacetone represented by formula (1): 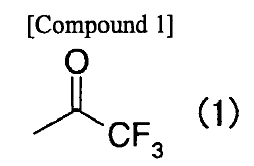
(a) a polynucleotide comprising the nucleotide sequence of any one of SEQ ID NOs: 1 to 8;
(b) a polynucleotide encoding a protein comprising the amino acid sequence of any one of SEQ ID NOs: 9 to 16;
(c) a polynucleotide encoding a protein comprising amino acids with one or more amino acid substitutions, deletions, insertions, and/or additions in the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
(d) a polynucleotide that hybridizes under stringent conditions with a DNA comprising the nucleotide sequence of any one of SEQ ID NOs: 1 to 8, wherein the polynucleotide encodes a protein having an activity of reducing 1,1,1-trifluoroacetone represented by formula (1) to produce (S)-1,1,1-trifluoro-2-propanol represented by formula (2); and
(e) a polynucleotide encoding a protein comprising an amino acid sequence having 80% or more homology to the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2).

2. A method for producing (R)-1,1,1-trifluoro-2-propanol represented by formula (3), 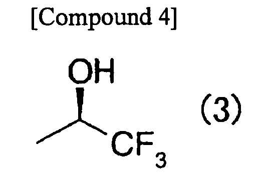 which comprises the step of reacting a protein encoded by the polynucleotide of any one of the following (a) to (e), a microorganism or a transformant strain that functionally expresses said protein, or a processed material thereof, with 1,1,1-trifluoroacetone represented by formula (1): 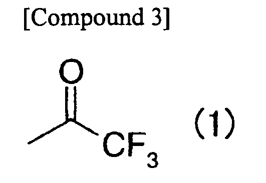
(a) a polynucleotide comprising the nucleotide sequence of SEQ ID NO: 17;
(b) a polynucleotide encoding a protein comprising the amino acid sequence of SEQ ID NO: 18;
(c) a polynucleotide encoding a protein comprising amino acids with one or more amino acid substitutions, deletions, insertions, and/or additions in the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
(d) a polynucleotide that hybridizes under stringent conditions with a DNA comprising the nucleotide sequence of SEQ ID NO: 17, wherein the polynucleotide encodes a protein having an activity of reducing 1,1,1-trifluoroacetone represented by formula (1) to produce (R)-1,1,1-trifluoro-2-propanol represented by formula (3); and
(e) a polynucleotide encoding a protein comprising an amino acid sequence having 80% or more homology to the amino acid sequence of SEQ ID NO: 18, wherein the protein has an activity of reducing 1,1,1-trifluoroacetone represented by formula (1) to produce (R)-1,1,1-trifluoro-2-propanol represented by formula (3).

3. A method for producing (S)-1,1,1-trifluoro-2-propanol represented by formula (2), 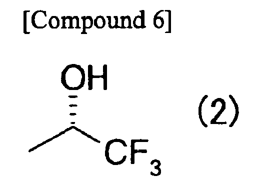 which comprises the step of reacting a protein encoded by the polynucleotide of any one of the following (a) to (e), a transformant strain that coexpresses a coenzyme corresponding to said protein and a dehydrogenase having an activity to regenerate reduced nicotinamide adenine dinucleotide (NADH) or reduced nicotinamide adenine dinucleotide phosphate (NADH), or a processed material thereof, with 1,1,1-trifluoroacetone represented by formula (1):
[Compound 5] 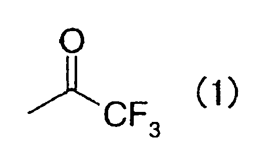
(a) a polynucleotide comprising the nucleotide sequence of any one of SEQ ID NOs: 1 to 8;
(b) a polynucleotide encoding a protein comprising the amino acid sequence of any one of SEQ ID NOs: 9 to 16;
(c) a polynucleotide encoding a protein comprising amino acids with one or more amino acid substitutions, deletions, insertions, and/or additions in the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2);
(d) a polynucleotide that hybridizes under stringent conditions with a DNA comprising the nucleotide sequence of any one of SEQ ID NOs: 1 to 8, wherein the polynucleotide encodes a protein having an activity of reducing 1,1,1-trifluoroacetone represented by formula (1) to produce (S)-1,1,1-trifluoro-2-propanol represented by formula (2); and
(e) a polynucleotide encoding a protein comprising an amino acid sequence having 80% or more homology to the amino acid sequence of any one of SEQ ID NOs: 9 to 16, wherein the protein has an activity of reducing 1,1,1-trifluoroacetone represented by formula (1) to produce (S)-1,1,1-trifluoro-2-propanol represented by formula (2).

4. A method for producing (R)-1,1,1-trifluoro-2-propanol represented by formula (3), 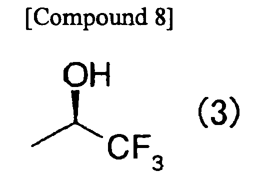 which comprises the step of reacting a protein encoded by the polynucleotide of any one of the following (a) to (e), a transformant strain that coexpresses a coenzyme corresponding to said protein and a dehydrogenase having an activity to regenerate reduced nicotinamide adenine dinucleotide (NADH) or reduced nicotinamide adenine dinucleotide phosphate (NADH), or a processed material thereof, with 1,1,1-trifluoroacetone represented by formula (1): 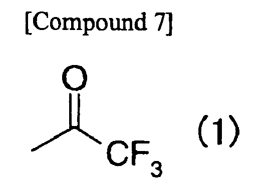
(a) a polynucleotide comprising the nucleotide sequence of SEQ ID NO: 17;
(b) a polynucleotide encoding a protein comprising the amino acid sequence of SEQ ID NO: 18;
(c) a polynucleotide encoding a protein comprising amino acids with one or more amino acid substitutions, deletions, insertions, and/or additions in the amino acid sequence of SEQ ID NO: 18, wherein the protein has an activity of reducing 1,1,1-trifluoroacetone represented by formula (1) to produce (R)-1,1,1-trifluoro-2-propanol represented by formula (3);
(d) a polynucleotide that hybridizes under stringent conditions with a DNA comprising the nucleotide sequence of SEQ ID NO: 17, wherein the polynucleotide encodes a protein having an activity of reducing 1,1,1-trifluoroacetone represented by formula (1) to produce (R)-1,1,1-trifluoro-2-propanol represented by formula (3); and
(e) a polynucleotide encoding a protein comprising an amino acid sequence having 80% or more homology to the amino acid sequence of SEQ ID NO: 18, wherein the protein has an activity of reducing 1,1,1-trifluoroacetone represented by formula (1) to produce (R)-1,1,1-trifluoro-2-propanol represented by formula (3).

5. The method of claim 3 or 4, wherein the dehydrogenase having an activity to regenerate reduced nicotinamide adenine dinucleotide (NADH) or reduced nicotinamide adenine dinucleotide phosphate (NADPH) is glucose dehydrogenase or formate dehydrogenase.

6. A method for stably producing (S)-1,1,1-trifluoro-2-propanol represented by formula (2) 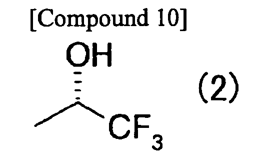 with an optical purity of 99.5% e.e. or more by reacting a protein encoded by the polynucleotide of any one of the following (a) to (e), a microorganism or a transformant strain that functionally expresses said protein, or a processed material thereof, with 1,1,1-trifluoroacetone represented by formula (1) within a pH range of 5.0 to 6.4: 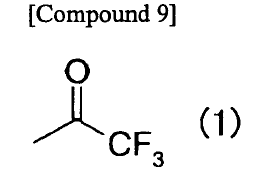
(a) a polynucleotide comprising the nucleotide sequence of SEQ ID NO: 1;
(b) a polynucleotide encoding a protein comprising the amino acid sequence of SEQ ID NO: 9;
(c) a polynucleotide encoding a protein comprising amino acids with one or more amino acid substitutions, deletions, insertions, and/or additions in the amino acid sequence of SEQ ID NO: 9, wherein the protein has an activity of reducing 1,1,1-trifluoroacetone represented by formula (1) to produce (S)-1,1,1-trifluoro-2-propanol represented by formula (2);
(d) a polynucleotide that hybridizes under stringent conditions with a DNA comprising the nucleotide sequence of SEQ ID NO: 1, wherein the polynucleotide encodes a protein having an activity of reducing 1,1,1-trifluoroacetone represented by formula (1) to produce (S)-1,1,1-trifluoro-2-propanol represented by formula (2); and
(e) a polynucleotide encoding a protein comprising an amino acid sequence having 80% or more homology to the amino acid sequence of SEQ ID NO: 9, wherein the protein has an activity of reducing 1,1,1-trifluoroacetone represented by formula (1) to produce (S)-1,1,1-trifluoro-2-propanol represented by formula (2).
